(19) Europäisches Patentamt
European Patent Office
Office européen des brevets

(11) EP 3 280 438 B1

(12) **EUROPEAN PATENT SPECIFICATION**

(45) Date of publication and mention of the grant of the patent:
**19.02.2020 Bulletin 2020/08**

(21) Application number: **16718476.1**

(22) Date of filing: **08.04.2016**

(51) Int Cl.:
***A61K 39/12*** *(2006.01)*

(86) International application number:
**PCT/IB2016/052008**

(87) International publication number:
**WO 2016/162845 (13.10.2016 Gazette 2016/41)**

(54) **RECOMBINANT LUMPY SKIN DISEASE VIRUS KNOCK-OUT MUTANT AND USES THEREOF**

KNOCKOUT-MUTANT DES REKOMBINANTEN VIRUS DER RINDER-KNÖTCHENKRANKHEIT UND VERWENDUNGEN DAVON

MUTANT INACTIVÉ DE VIRUS DE DERMATOSE NODULAIRE RECOMBINANT ET SES UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.04.2015 GB 201506004**

(43) Date of publication of application:
**14.02.2018 Bulletin 2018/07**

(73) Proprietor: **Agricultural Research Council**
**Pretoria**
**0083 (ZA)**

(72) Inventors:
- **WALLACE, David Brian**
  **0182 Pretoria (ZA)**
- **MATHER, Arshad Saleh**
  **0183 Centurion (ZA)**
- **KARA, Pravesh Deepak**
  **0037 Pretoria (ZA)**

(74) Representative: **Appleyard Lees IP LLP**
**15 Clare Road**
**Halifax HX1 2HY (GB)**

(56) References cited:
- **KARA P D ET AL: "Comparative sequence analysis of the South African vaccine strain and two virulent field isolates of Lumpy skin disease virus", ARCHIVES OF VIROLOGY, SPRINGER WIEN, AT, vol. 148, 1 June 2003 (2003-06-01), pages 1335-1356, XP008165302, ISSN: 0304-8608**
- **P. OUYANG ET AL: "IL-10 encoded by viruses: a remarkable example of independent acquisition of a cellular gene by viruses and its subsequent evolution in the viral genome", JOURNAL OF GENERAL VIROLOGY., vol. 95, no. Pt_2, 1 February 2014 (2014-02-01), pages 245-262, XP055280338, GB ISSN: 0022-1317, DOI: 10.1099/vir.0.058966-0**
- **E. R. TULMAN ET AL: "Genome of Lumpy Skin Disease Virus", JOURNAL OF VIROLOGY., vol. 75, no. 15, 1 August 2001 (2001-08-01), pages 7122-7130, XP055280347, US ISSN: 0022-538X, DOI: 10.1128/JVI.75.15.7122-7130.2001**
- **WALLACE D B ET AL: "Immune responses to recombinants of the South African vaccine strain of lumpy skin disease virus generated by using thymidine kinase gene insertion", VACCINE, ELSEVIER LTD, GB, vol. 23, no. 23, 27 April 2005 (2005-04-27), pages 3061-3067, XP027652411, ISSN: 0264-410X [retrieved on 2005-04-27]**
- **ASPDEN K ET AL: "Evaluation of lumpy skin disease virus, a capripoxvirus, as a replication-deficient vaccine vector", JOURNAL OF GENERAL VIROLOGY, SOCIETY FOR GENERAL MICROBIOLOGY, SPENCERS WOOD, GB, vol. 84, no. Pt 8, 1 August 2003 (2003-08-01), pages 1985-1996, XP002544352, ISSN: 0022-1317, DOI: 10.1099/VIR.0.19116-0**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 280 438 B1

- HANI BOSHRA ET AL: "A lumpy skin disease virus deficient of an IL-10 gene homologue provides protective immunity against virulent capripoxvirus challenge in sheep and goats", ANTIVIRAL RESEARCH, vol. 123, 1 November 2015 (2015-11-01), pages 39-49, XP055280318, NL ISSN: 0166-3542, DOI: 10.1016/j.antiviral.2015.08.016

## Description

## BACKGROUND OF THE INVENTION

This invention relates to a live-attenuated recombinant lumpy skin disease virus knock-out mutant, wherein the interleukin-10-like gene has been inactivated in the viral genome. The invention specifically relates to the recombinant lumpy skin disease virus knock-out mutant, pharmaceutical compositions comprising the knock-out mutant and use of the knock-out mutant.

Lumpy skin disease (LSD) is an acute or inapparent disease of cattle caused by lumpy skin disease virus (LSDV), a poxvirus, belonging to the genus *Capripoxvirinae*. It is an important, insect-borne infectious disease of cattle; and is endemic in most African countries, and it has also spread to the Middle East and Turkey.

Poxviruses infecting vertebrates belong to the family Poxviridae, subfamily Chordopoxvirinae. They are large double-stranded DNA viruses that replicate in the cytoplasm. They contain a central coding region flanked by inverted terminal repeat (ITR) regions that are identical but oppositely orientated sequences at the two ends of the genome and are found in all poxviruses examined (Esposito and Knight, 1985). The large, conserved central region predominantly contains genes essential for virus structure and replication, while terminally located genes tend to be more variable expressing genes involved in host range restriction and immunomodulation (Esposito and Knight, 1985).

Poxviruses are known to encode a multitude of gene products that allow them to evade, obstruct or subvert key components of the host innate and acquired immune responses. There is, however not one immunomodulatory gene that is present in all poxviruses, indicating that virus pathogenesis may be influenced by different mechanisms depending on the specific virus-host interactions at play. Individual gene knockouts have been shown to partially attenuate viruses. The vast array of these immunomodulatory or virulence genes act in concert to enable the virus to evade or suppress the host immune responses following infection.

The strategies used by poxviruses in infecting their hosts allows insight into diverse possibilities and developments that have and can lead to the design of new vaccines, as well as possible therapeutic tools for the treatment of diseases.

The purpose of the invention was to generate an improved live-attenuated LSDV vaccine using gene knockout technology targeting a putative LSDV immunomodulatory gene such as the interleukin-10-like gene. This LSDV knockout virus would be evaluated for use as a vaccine to protect cattle against LSD challenge, sheep against sheeppox challenge and goats against goatpox challenge.

The virulent LSDV Warmbaths field isolate was chosen as the backbone to generate this virus/vaccine as it was able to stimulate host immune responses. The LSDV OP vaccine was considered for use as the backbone but due to it being already attenuated, the removal of such a gene may reduce its ability to stimulate relevant immune responses.

Since capripoxviruses, which include sheeppox and goatpox viruses, share a common major antigen (P32) neutralising site, the LSDV-based vaccine has the potential to protect sheep and goats against sheep pox and goat pox.

The generation of a knockout virus has a number of potential outcomes such that it is either attenuated enough to produce less severe symptoms and is able to provide animals protection against infection. It may also have a negative outcome by generating a more virulent virus producing severe disease.

LSDV is categorised as an OIE notifiable disease. The disease does not usually have a high mortality rate, but is of economic importance as it causes decreased milk production, temporary or permanent infertility in cows and bulls, abortions and damage to hides. There are however times when the disease does cause death due to emaciation and secondary bacterial infections.

Vaccination with an attenuated strain of lumpy skin disease virus (LSDV) is the only viable means of LSD control in endemic areas such as South Africa and other parts of Africa.

There are six capripoxvirus vaccines that are in use specifically for the control of LSD. These are a Kenyan sheep-and-goat pox (KS-1), Yugoslavian RM 65 sheep pox strain, Romanian sheep pox strain, the South African Onderstepoort LSDV Neethling strain, vaccine (LSDV OBP) that is sold by Onderstepoort Biological products, Lumpyvax™ and Herbivac®LS. All capripoxviruses share a major common antigen neutralising site, so that different strains of capripoxvirus, for example sheep pox virus, can afford cross protection of cattle against LSD.

The SA LSDV OBP vaccine strain, developed in 1968 by passage of a field isolate in tissue culture and on the chorioallantoic membranes of embryonated hen's eggs (van Rooyen et al., 1969) is currently in use in South Africa. There are two other LSDV vaccines that have been developed in South Africa. These are Lumpyvax™, a live attenuated virus (SIS type) and Herbivac®LS, a live attenuated virus (modified Neethling type) marketed by Intervet and Deltamune respectively. There is however no further information on these vaccines due to intellectual property protection rights so we are limited in our understanding of their development and efficacy.

Although vaccination against LSD used to be considered to provide life-long protection (Weiss, 1968), problems of "vaccine failure" have been reported, such as during the 1990-1991 LSD outbreak, as well as from subsequent outbreaks (Hunter and Wallace, 2001). These problems may have been due to prevailing climatic conditions favouring the reproduction and spread of insect vectors (Hunter and Wallace, 2001). Low levels of vaccine use during this period and in subsequent years, may also contribute to the spread of LSD (Hunter and Wallace, 2001). Other problems may include a shorter period of induced immunity and most importantly, there is no level of detectable antibody production in 10% of dams after vaccination, surmised to leave calves susceptible to infection (Hunter and Wallace, 2001). Annual vaccination against LSD is thus recommended.

The molecular characterisation of both the LSDV Neethling strain vaccine (GenBank accession No: AF409138.1) and the LSDV Warmbaths field isolate (GenBank accession No: AF409137.1) by our group, provided us the opportunity to identify genes putatively involved in host-range, virulence and immunomodulation or inhibition of the host responses to infection (Kara, et al. 2003). Among these are genes for an interleukin-10-like protein, interleukin-1 receptor-like protein, apoptosis regulator, interferon-gamma like receptor, interleukin-18 binding protein, G protein-coupled CC chemokine receptor, interferon-alpha/beta binding protein, and an OX-2 like protein.

These putative immunomodulatory genes have been removed or knocked-out in certain poxviruses such as vaccinia virus (VV), myxoma virus (MYX) and the parapox, orf virus (ORFV), as well as other poxviruses. By knocking them out it provides a tool to functionally characterise them, allowing for a better understanding of mechanisms of pathogenesis. A number of these knockout recombinant viruses have been evaluated in animal models with some of them exhibiting various degrees of attenuation.

Sheep pox (SPP) and goat pox (GTP) are also primary viral diseases affecting the small ruminant livestock industry in parts of Asia and Africa. Sheep pox virus (SPPV) and goat pox virus (GTPV), along with lumpy skin disease virus (LSDV), are all members of the viral genus *Capripoxvirinae.* In naive animals, both SPPV and GTPV can induce morbidity and mortality rates as high as 100% and approaching 90% depending on the isolate, respectively. For lumpy skin disease in cattle the results for both field outbreaks and experimental infection are highly variable, with morbidity ranging from 3%-85% and mortality rates being generally less than 3%. Due to the severity of all three diseases, along with their potential negative effects on local economies, the Office International des Epizooties has designated them as listed diseases (OIE, 2010). Capripoxviruses share a high degree of sequence homology, and immune responses induced after infection are generally cross-protective, as supported in one early study in which a degree of cross-protection for all three CaPVs was demonstrated in sheep, goats and cattle (Capstick, 1961).

Following a 4-8 day incubation period, SPPV and GTPV infection is characterized by fever, increases in internal body temperature and respiration rate, and shortly thereafter the appearance of macules in the skin, which ultimately develop into pox lesions (Bhanuprakash et al., 2006; Babiuk et al., 2008a). These lesions, along with increased oral and nasal secretions, contain high viral loads, and serve as primary routes for contact transmission (Bowden et al., 2008).

Currently, commercial vaccines are available to control outbreaks of these diseases in endemic countries. While differing in geographical origin, they share one common trait; that is, each vaccine was derived from a previously virulent strain and serially passaged in cell culture until attenuated (Babiuk et al., 2008a; Davies and Mbugwa, 1985; Kitching, 2003). Although field studies have generally shown these vaccines to have a high degree of efficacy, little is known about the precise mechanism/s of attenuation.

This invention relates to a virulent LSDV strain which has been attenuated through the disruption of its open reading frame 005 (ORF005), coding for a putative virulence factor with homology to interleukin-10 (IL-10) using homologous recombination. This attenuated strain was evaluated in cattle, sheep and goats for safety and efficacy against virulent capripoxvirus isolates.

Kara P D et al: "Comparative sequence analysis of the South African vaccine strain and two virulent field isolates of Lumpy skin disease virus", Archives of Virology, vol. 148, pages 1335-1356 discloses a sequence comparison between a virulent LSDV strain and a corresponding highly attenuated vaccine strain. Of the 156 open reading frames identified as being common to both the attenuated and virulent LSDV strains, 114 had amino acid differences. The IL-10-like ORF comprises two mutations which potentially affect its function. This document further suggests that the mutation of a few specific proteins may be sufficient to obtain an attenuated strain. However, Kara et al does not refer to a LSDV strain in which a knock-out of the IL-10-like gene has been generated by deleting the IL-10-like gene.

Ouyang P et al: "IL-10 encoded by viruses: a remarkable example of independent acquisition of a cellular gene by viruses and its subsequent evolution in the viral genome", Journal of General Virology., vol. 95, pages 245-262 is a review dealing with IL-10 encoded by viruses and mentions that IL-10-like genes have been reported in 19 viruses including LSDV. Experimental results suggest that IL-10-like is involved in immunosuppression and that in some cases could be an appropriate target to generate attenuated recombinant vaccines. In Ouyang et al the in vivo function of IL-10-like proteins was studied via the generation of knock-out mutants in three viruses RhCMV, ORFV and CyHV-3. In two of these viruses, RhCMV and ORFV, the deletion of the IL-10-like gene had some influence on the virulence of the strain but it is not shown that the corresponding strains would be suitable as attenuated vaccine strains. In the third strain, CyHV-3, the deletion of the IL-10-like gene does not result in any attenuation of the strain. Thus, Ouyang et al does not teach an LSDV strain having an IL-10-like knock-out or provide any evidence whether such a strain would be suitable as attenuated vaccine against LSDV, sheeppox and goatpox.

## SUMMARY OF THE INVENTION

The present invention is defined in the appended claims.

Also disclosed is a live-attenuated recombinant lumpy skin disease virus knock-out mutant, wherein the interleukin-10-like gene has been inactivated in the viral genome, pharmaceutical compositions comprising the knock-out mutant, methods of producing the knock out mutant and use of the knock-out mutant in the treatment of disease.

According to a first aspect there is provided for a lumpy skin disease virus (LSDV) knock-out mutant, wherein the interleukin-10-like gene has been inactivated in the viral genome by deletion. In a preferred embodiment, the interleukin-10-like gene is deleted through homologous recombination.

It will be appreciated that inactivation of the interleukin-10-like gene results in the production of a live attenuated lumpy skin disease virus strain. It will further be appreciated that the interleukin-10-like gene is situated in open reading frame 005 (ORF005) of virulent lumpy skin disease virus strains.

A second aspect provides for a pharmaceutical composition, comprising a lumpy skin disease virus knock-out mutant, wherein the interleukin-10-like gene has been deleted from the viral genome, together with a pharmaceutically acceptable diluent and/or excipient. The pharmaceutical composition may further include an adjuvant.

The pharmaceutical composition is capable of eliciting a protective immune response in an animal against a disease caused by a virus from the viral genus *Capripoxvirinae.* Non-limiting embodiments of the disease include lumpy skin disease, sheep pox and goat pox, however those of skill in the art will appreciate that the pharmaceutical composition may also be effective against other diseases caused by viruses of the genus *Capripoxvirinae,* as yet undiscovered/undescribed.

In one embodiment the animal is a ruminant. Preferably, the ruminant is selected from the group consisting of, but not limited to, cattle, sheep, goats and wild ungulates.

In a further embodiment the pharmaceutical composition is formulated for intramuscular, intradermal, intravenous, intraperitoneal or subcutaneous, oral or sublingual administration. It will be appreciated that the pharmaceutical composition may be formulated for administration to the animal in a single dose, alternatively the pharmaceutical composition may be formulated for administration to the animal in two or more doses.

A further aspect provides of a method of producing a live, attenuated LSDV knock-out mutant, wherein the method comprises the steps of (i) infecting a host cell with a parental LSDV strain, (ii) transfecting the host cell with an insertion vector containing a selection marker, and (iii) selecting recombinant LSDV knock-out mutants. It will be appreciated that the parental LSDV strain may be a wild-type, attenuated, inactivated strain or bacterial artificial chromosome (BAC) clone.

A fourth aspect provides for the use of a LSDV knock-out mutant for the manufacture of a medicament for use in a method of preventing a disease in an animal wherein the disease is caused by a virus from the viral genus *Capripoxvirinae.* More preferably, the disease is selected from lumpy skin disease, sheep pox and goat pox.

A fifth aspect provides for a LSDV knock-out mutant for use in a method of preventing a disease in an animal wherein the disease is caused by a virus from the viral genus *Capripoxvirinae.* More preferably, the disease is selected from lumpy skin disease, sheep pox and goat pox. It will be appreciated that the animal may be a ruminant. Preferably, the ruminant is selected from the group consisting of, but not limited to, cattle, sheep, goats and wild ungulates.

A sixth aspect provides for an insertion vector for obtaining or producing a lumpy skin disease virus (LSDV) knock-out mutant, wherein the insertion vector inactivates an interleukin-10-like gene to produce the knock-out mutant.

In one embodiment the interleukin-10-like gene of an LSDV parental strain is inactivated by deletion from the viral genome upon transfection of a cell with the insertion vector.

## BRIEF DESCRIPTION OF THE FIGURES

Non-limiting embodiments of the invention will now be described by way of example only and with reference to the following figures:

> **Figure 1:** Universal cloning vector pUL for use in the construction of a transfer vector. The vector consists of unique restriction sites on either side of an enhanced green fluorescent protein (EGFP) reporter gene (under control of the vaccinia virus (VV) P11 promoter), and the *E. coli* xanthine-guanine phosphoribosyl-transferase (gpt) dominant selectable marker gene under control of the VV P7.5 promoter. An additional VV P7.5 promoter is situated downstream of this, to permit subsequent deletion of the *E.coli* gpt gene. The unique restriction sites on either side of the EGFP and gpt genes allows for the insertion of sequences that are homologous to the sequence of the gene/s to be removed or disrupted. Arrows indicate the direction/orientation of the genes and promoters.
>
> **Figure 2:** LSDV insertion vector, pTW005, for use in the generation of recombinant LSDV WB005KO. The vector consists of the LSDV ORF 005 gene disrupted by the EGFP reporter gene (under control of the VV P11 promoter), and the *E. coli* gpt dominant selectable marker gene (under control of the VVP7.5 promoter, with an additional VV P7.5 promoter downstream of this, to permit subsequent deletion of the *E.coli* gpt gene). Arrows indicate the direction/orientation of the genes and promoters.

**Figure 3:** Clinical signs observed in calves following vaccination with cell culture material (Group 1, negative control), the LSDV OBP vaccine (Group 2) and the LSDV WB005KO construct (Group 3).

**Figure 4:** Neutralising antibody titres in the sera of calves at various time points following vaccination with cell culture material (Group 1, negative control), the LSDV OBP vaccine (Group 2) and the LSDV WB005KO construct (Group 3). Neutralising titres are presented as mean log values.

**Figure 5**: Proliferative responses of PBMCs stimulated with 1 x $10^5$ pfu/ml of the LSDV WB antigen, of animals vaccinated with cell culture medium (Group 1), LSDV OBP vaccine (Group 2) and the LSDV WB005KO construct (Group 3). The results were expressed as the stimulation index (SI) which is defined as the mean counts per minute of the LSDV Warmbaths antigen divided by the mean counts per minute (cpm) of the unstimulated PBMCs. A response was considered positive if the result was two times higher than the SI of the negative control with a significant p-value (p-value $\leq$ 0.01, as determined by Student's t-test).

**Figure 6:** An example of the crust-like lesions observed in both Group 1 (negative control) and Group 2 (LSDV OBP vaccine) calves following challenge with the LSDV Warmbaths (LSDV WB) isolate.

**Figure 7:** Neutralising antibody titres in the sera of calves from Groups 1, 2 and 3 at various times following challenge with the LSDV WB isolate. Neutralising titres are presented as mean log values.

**Figure 8:** Proliferative responses of PBMCs stimulated with 1 x $10^5$ pfu/ml of the LSDV WB antigen, from calves in groups 1, 2 and 3 following challenge with the LSDV WB isolate. The results were expressed as the stimulation index (SI) which is defined as the mean counts per minute of the LSDV Warmbaths antigen divided by the mean counts per minute (cpm) of the unstimulated PBMCs. A response was considered positive if the result was two times higher than the SI of the negative control with a significant p-value (p-value $\leq$ 0.01, as determined by Student's t-test).

**Figure 9:** Rectal temperatures of sheep and goats following infection/challenge with virulent capripoxviruses. Rectal temperatures of both vaccinated/unvaccinated sheep and goats were measured prior to viral challenge, and at regular daily intervals following challenge. Results are presented as mean temperatures of each group, with standard deviations indicated at each time point.

**Figure 10:** Clinical signs and gross pathology of unvaccinated/vaccinated sheep and goats following capripoxvirus challenge at 10 dpc. (A) Conjunctivitis in unvaccinated sheep; (B, C) the lack of pox lesions in vaccinated sheep and goats, respectively, are in contrast to pox lesions seen in unvaccinated goats (D) and sheep (F) at the same time point. Nasal and mucosal discharges were also observed in unvaccinated animals (E).

**Figure 11:** Quantification of capripoxvirus DNA in blood, as determined by real-time PCR. Whole blood was obtained from vaccinated and unvaccinated sheep and goats at multiple time points over 20 (goat) or 21 (sheep) dpv. Viral copies at each time point are presented as mean values with standard deviations. Differences between unvaccinated and vaccinated animals were significant ($P<0.05$) as determined by t-test.

**Figure 12:** Seroconversion of vaccinated/unvaccinated sheep and goats. Capripoxvirus-specific IgG antibody titres in sheep (A) and goats (B) were measured using an indirect ELISA following vaccination (days 0-21) and following viral challenge with virulent capripoxviruses (days 22-42). Results are presented as mean values with standard deviations at each time point.

**Figure 13:** Quantification of capripoxvirus-specific neutralising antibodies in vaccinated/unvaccinated sheep (A) and goats (B). Virus neutralisation tests (VNT) were performed using sera obtained following vaccination and capripoxvirus challenge at various time points. VNT titres are presented as mean log values with standard deviations at each time point.

**Figure 14:** Quantification of interferon-gamma (IFN-$\gamma$) secretion in samples from vaccinated and unvaccinated goats at 14 and 21 dpv. Measurement of IFN-$\gamma$ levels were performed using an IFN-$\gamma$ ELISA and cross-reactive antibodies against bovine IFN-$\gamma$, and quantified using a standard provided by the manufacturer. Results are the mean values with standard deviations. Differences in IFN-$\gamma$ levels in unvaccinated and vaccinated goats were significant ($P<0.05$) as determined by t-test.

## DETAILED DESCRIPTION OF THE INVENTION

The present invention will now be described more fully hereinafter with reference to the accompanying drawings, in which some, but not all embodiments of the invention are shown.

The invention as described should not be limited to the specific embodiments disclosed and modifications and other embodiments are intended to be included within the scope of the invention. Although specific terms are employed herein, they are used in a generic and descriptive sense only and not for purposes of limitation.

As used throughout this specification and in the claims which follow, the singular forms "a", "an" and "the" include the plural form, unless the context clearly indicates otherwise.

The terminology and phraseology used herein is for the purpose of description and should not be regarded as limiting. The use of the terms "comprising", "containing", "having" and "including" and variations thereof used herein, are meant to encompass the items listed thereafter and equivalents thereof as well as additional items.

By "condition associated with poxvirus infection" is meant any condition, disease or disorder that has been correlated with the presence of an existing poxvirus infection, it includes secondary effects, such as reductions in milk production, temporary or permanent infertility in cows and bulls, abortions and damage to hides, weight loss and wool brake.

The organism according to the invention includes, without limitation, a lumpy skin disease virus knock-out mutant, wherein the interleukin-10-like gene has been inactivated in the viral genome by deletion.

The present invention has targeted the interleukin-10-like gene which is situated in open reading frame 005 (ORF005) of the LSDV Warmbaths field isolate (GenBank Accession No. AF409137.1). SEQ ID NO:9 provides a representative sequence of the flanking regions of ORF005 together with ORF005 containing the interleukin-10-like gene in the LSDV Warmbaths field isolate. SEQ ID NO:10 provides a representative sequence of the same region of the lumpy skin disease virus (LSDV) knock-out mutant showing the two flanking regions of ORF005, together with the EGFP marker gene sequence that has been inserted into the LSDV Warmbaths field isolate, replacing most of the ORF005 gene sequence, resulting in the generation of the knock-out mutant.

The term "recombinant" means that something has been recombined. When used with reference to a nucleic acid construct the term refers to a molecule that comprises nucleic acid sequences that are joined together or produced by means of molecular biological techniques. Recombinant nucleic acid constructs may include a nucleotide sequence which is ligated to, or is manipulated to become ligated to, a nucleic acid sequence to which it is not ligated in nature, or to which it is ligated at a different location in nature. Accordingly, a recombinant nucleic acid construct indicates that the nucleic acid molecule has been manipulated using genetic engineering, i.e. by human intervention. Recombinant nucleic acid constructs may be introduced into a host cell by infection or transfection. Such recombinant nucleic acid constructs may include sequences derived from the same host cell species or from different host cell species.

The lumpy skin disease virus knock-out mutants or compositions of the invention can be provided either alone or in combination with other compounds (for example, nucleic acid molecules, small molecules, peptides, or peptide analogues), in the presence of a liposome, an adjuvant, or any carrier, such as a pharmaceutically acceptable carrier and in a form suitable for administration to mammals, for example, humans, cattle, sheep, etc.

As used herein a "pharmaceutically acceptable carrier" or "excipient" includes any and all antibacterial and antifungal agents, coatings, dispersion media, solvents, isotonic and absorption delaying agents, and the like that are physiologically compatible. A "pharmaceutically acceptable carrier" may include a solid or liquid filler, diluent or encapsulating substance which may be safely used for the administration of the lumpy skin disease virus knock-out mutant or vaccine composition to a subject. The pharmaceutically acceptable carrier can be suitable for intramuscular, intradermal, intravenous, intraperitoneal, subcutaneous, oral or sublingual administration. Pharmaceutically acceptable carriers include sterile aqueous solutions, dispersions and sterile powders for the preparation of sterile solutions. The use of media and agents for the preparation of pharmaceutically active substances is well known in the art. Where any conventional media or agent is incompatible with the active compound, use thereof in the pharmaceutical compositions of the invention is not contemplated. Supplementary active compounds can also be incorporated into the compositions.

Suitable formulations or compositions to administer the lumpy skin disease virus knock-out mutants and compositions to subjects suffering from poxvirus infection or subjects which are presymptomatic for a condition associated with poxvirus infection fall within the scope of the invention. Any appropriate route of administration may be employed, such as, parenteral, intravenous, subcutaneous, intramuscular, intracranial, intraorbital, ophthalmic, intraventricular, intracapsular, intraspinal, intrathecal, intracistemal, intraperitoneal, intranasal, aerosol, topical, or oral administration.

As used herein the term "subject" includes both wild and domestic ruminants. Preferably, the subjects are cattle, sheep and/or goats.

For vaccine formulations and pharmaceutical compositions, an effective amount of the lumpy skin disease virus knock-out mutants or compositions of the invention can be provided, either alone or in combination with other compounds, with immunological adjuvants, for example, aluminium hydroxide dimethyldioctadecyl-ammonium hydroxide or Freund's incomplete adjuvant. The lumpy skin disease virus knock-out mutants or compositions of the invention may also be linked with suitable carriers and/or other molecules, such as bovine serum albumin or keyhole limpet haemocyanin in order to enhance immunogenicity.

In some embodiments, the lumpy skin disease virus knock-out mutants or compositions according to the invention may be provided in a kit, optionally with a carrier and/or an adjuvant, together with instructions for use.

An "effective amount" of a compound according to the invention includes a therapeutically effective amount, immunologically effective amount, or a prophylactically effective amount. A "therapeutically effective amount" refers to an amount effective, at dosages and for periods of time necessary, to achieve the desired therapeutic result, such as treatment of an infection or a condition associated with such infection. The outcome of the treatment may for example be measured by a decrease in viraemia, inhibition of viral gene expression, delay in development of a pathology associated with poxvirus infection, stimulation of the immune system, or any other method of determining a therapeutic benefit. A therapeutically effective amount of a compound may vary according to factors such as the disease state, age, sex, and weight of the individual, and the ability of the compound to elicit a desired response in the individual. Dosage regimens may be adjusted to provide the optimum therapeutic response. A therapeutically effective amount is also one in which any toxic or detrimental effects of the compound are outweighed by the therapeutically beneficial effects.

The dosage of any of the lumpy skin disease virus knock-out mutants or compositions of the present invention will vary depending on the symptoms, age and body weight of the subject, the nature and severity of the disorder to be treated or prevented, the route of administration, and the form of the composition. Any of the compositions of the invention may be administered in a single dose or in multiple doses. The dosages of the compositions of the invention may be readily determined by techniques known to those of skill in the art or as taught herein.

By "immunogenically effective amount" is meant an amount effective, at dosages and for periods of time necessary, to achieve a desired immune response. The desired immune response may include stimulation or elicitation of an immune response, for instance a T-cell response.

A "prophylactically effective amount" refers to an amount effective, at dosages and for periods of time necessary, to achieve a desired prophylactic result, such as prevention of onset of a condition associated with poxvirus infection. Typically, a prophylactic dose is used in subjects prior to or at an earlier stage of disease, so that a prophylactically effective amount may be less than a therapeutically effective amount.

Dosage values may vary and be adjusted over time according to the individual need and the judgment of the person administering or supervising the administration of the lumpy skin disease virus knock-out mutants or compositions of the invention. Dosage ranges set forth herein are exemplary only and do not limit the dosage ranges that may be selected. The amount of active compound(s) in the composition may vary according to factors such as the disease state, age, sex, and weight of the individual. Dosage regimens may be adjusted to provide the optimum therapeutic response. For example, a single dose may be administered, or multiple doses may be administered over time. It may be advantageous to formulate the compositions in dosage unit forms for ease of administration and uniformity of dosage.

The term "preventing", when used in relation to an infectious disease, or other medical disease or condition, is well understood in the art, and includes administration of a composition which reduces the frequency of or delays the onset of symptoms of a condition in a subject relative to a subject which does not receive the composition. Prevention of a disease includes, for example, reducing the number of diagnoses of the infection in a treated population versus an untreated control population, and/or delaying the onset of symptoms of the infection in a treated population versus an untreated control population.

The term "prophylactic or therapeutic" treatment is well known to those of skill in the art and includes administration to a subject of one or more of the compositions of the invention. If the composition is administered prior to clinical manifestation of the unwanted condition (e.g., disease or other unwanted state of the subject) then the treatment is prophylactic, i.e., it protects the host against developing the unwanted condition, whereas if it is administered after manifestation of the unwanted condition, the treatment is therapeutic (i.e., it is intended to diminish, ameliorate, or stabilise the existing unwanted condition or side effects thereof).

Toxicity and therapeutic efficacy of compositions of the invention may be determined by standard pharmaceutical procedures in cell culture or using experimental animals, such as by determining the $LD_{50}$ and the $ED_{50}$. Data obtained from the cell cultures and/or animal studies may be used to formulate a dosage range for use in a subject. The dosage of any composition of the invention lies preferably within a range of circulating concentrations that include the $ED_{50}$ but which has little or no toxicity. The dosage may vary within this range depending upon the dosage form employed and the route of administration utilised. For compositions of the present invention, the therapeutically effective dose may be estimated initially from cell culture assays.

The invention also relates in part to a method of prophylactically treating a subject, comprising administering to the subject in need thereof a prophylactically effective amount of the lumpy skin disease virus knock-out mutants or compositions of the present invention.

The following examples are offered by way of illustration and not by way of limitation.

**EXAMPLE 1**

**Construction of the universal cloning vector**

A universal cloning vector pUL (Figure 1) was constructed. Briefly, the pMOS vector was used as the backbone for constructing the universal cloning vector. The vector pHGS7-E was digested with *Mlu*I and *Xmn*I to obtain the late vaccinia virus (W) promoter P11 together with the enhanced green fluorescent protein (EGFP) gene. The EGFP gene is controlled by the W P11 promoter. This fragment was inserted in the *Xho*I site of the pARO-gpt vector. The plasmid was named pAgE. The pHGS7-E and pARO-gpt plasmids were obtained from D. Wallace. All restriction enzymes were purchased from Fermentas.

The pAgE plasmid was digested with *Bam*HI to remove both the W P11 promoter andEGFP together with the W early-late P7.5 promoter that controls the xanthine-guanine phosphoribosyl-transferase (gpt) gene. This fragment was then inserted into the *Bam*HI site of the pMOSBlue plasmid. The plasmid was named pMgE.

The LSDV Warmbaths field isolate (GenBank Accession No. AF409137.1) was used as template for PCR to amplify the left flanking sequence of ORF005 with primers 005-KOL-F (forward) (5'-AAT AAC CCT GCA GGC TTA AGT CAT GAA TGG AAA ACA AAC ACA AAA ATA ATA C-3' (SEQ ID NO:1) and 005-KOL-R (reverse) (5'-TAA ACT GCA GAC GCG TTG ATC ACT TAA GCA ATG TAA AAA ATC ACA CGA TAT G-3') (SEQ ID NO:2). Restriction enzyme recognition sequences were also included in the primer sequences to facilitate the subsequent insertion into pMgE (restriction enzyme recognition sequences *Sda*I, *Bsp*TI and *Bsp*HI were added for 005-KOL-F and *Pst*I, *Mlu*I, *Bcl*I and *Bsp*TI for 005-KOL-R).

The PCR fragment (named 005KOL) was inserted upstream of the EGFP and gpt genes into the *Sse*8387I and *Pst*I sites of the pMgE plasmid, and plasmid pMgE-005-L was generated and selected. The plasmid pARO-VP7 (obtained from D Wallace), containing the VV P7.5 promoter and the VP7 gene from African horse sickness virus (AHSV), was digested with NdeI and EcoRI to remove the P7.5 promoter and VP7 gene fragment, which were then inserted downstream of the EGFP and gpt genes in the NotI site of plasmid pMgE-005-L to obtain plasmid pMgE-005L-VP7. The AHSV VP7 gene was removed by digesting the plasmid with BamHI, leaving behind the W P7.5 promoter. The 005KOL fragment was removed by digesting with BspHI, resulting in the final universal cloning vector, pUL, (Figure 1). This vector was used to construct the insertion vector, pTW005

**Construction of the insertion vector (pTW005)**

The left and right flanking fragments of ORF005 were amplified using the LSDV WB isolate as the template (Figure 2). PCR was performed using TaKaRa Ex Taq™ DNA Polymerase (Takara, Japan) according to manufacturer's guidelines. Primer pair LF005FWD (5'-ATA CAT CTT AAG CAG TTT TAA TCG CTC TAA AAT TC-3') (SEQ ID NO:3) and LF005REV (5'-TAG TAG ACG CGT GTA ATC AAA GCT AAC GTC GTC AC-3') (SEQ ID NO:4) were used to amplify the left flanking sequence of ORF005, namely 005L. Primer pair LF005FWD and LF005REV have *Bsp*TI and *Mlu*I restriction recognition sites at their five-prime ends, respectively.

Primer pair RT005FWD (5'-TCC TCC CCC GGG GAA AAG TAC ATA GTT AAA AAG TAA TC-3') (SEQ ID NO:5) and RT005REV (5'-ATC GAG CTC TAT CGT TTG TAA GAA ATT ATA ACG-3') (SEQ ID NO:6) were used to amplify the right flanking sequence of ORF005, namely 005R. Primer pair RT005FWD and RT005REV have *Sma*I/*Ava*I and *Sac*I restriction recognition sites at their five-prime ends, respectively.

PCR was carried out with an initial denaturing step at 98°C for 5 minutes, followed by 35 cycles at 98°C for 1 minute, 58°C for 30 seconds, 72°C for 1 minute and a final elongation step at 72°C for 10 minutes. The samples were then subjected to agarose gel electrophoresis using a 1.2% agarose gel for confirmation. The remaining 005L and 005R gene fragment amplification products were electrophoresed using a 0.8% agarose gel and the fragments were recovered using a sterile scalpel blade and purified using a Qiagen® gel extraction purification kit (Qiagen®, Germany).

The purified fragments were then inserted into the pGEMT-Easy vector (Promega, USA) according to the manufacturer's instructions, giving rise to plasmids pGTE-005L and pGTE-005R. Plasmid pGTE-005L was digested with *Bsp*TI and *Mlu*I, and inserted upstream of the EGFP and gpt genes, while pGTE-005R was digested with *Ava*I and *Sac*I and inserted downstream of the EGFP and gpt genes giving rise to pTW005 (Figure 2). The final insertion vector pTW005 (Figure 2) was used in the generation of the recombinant LSDV IL-10 like gene (ORF005) knock out construct using the MPA-selection approach in which the transfections were performed using the jetPEI® transfection reagent (Polyplus transfection, France).

**Primary FBT/FCE cell preparation**

Foetal bovine testes (FBT) and foetal calf ear (FCE) tissue was obtained from the Strydfontein abattoir close to Pretoria North, South Africa. The testes were sliced open to remove the capsules, blood vessels and fat. The soft testes tissue was then removed and cut into smaller pieces (0.5 cm x 0.5 cm cubes). The FCE tissue (obtained from foetuses less that 30cm in length) was also cut into small pieces as above. The FBT tissue and FCE tissue were then washed with phosphate buffered saline (PBS) containing 1X antibiotics (penicillin, streptomycin and amphotericin B) (Lonza, USA) to rinse off excess blood. The chopped tissues were incubated separately with 1% Dispase (Roche®) at room temperature for 30 minutes. Fresh 1% Dispase was added to the remaining tissue and the process repeated. The trypsinzed mixture was then pooled, and 8% foetal calf serum (FCS) (Gibco/Lonza) was added. The mixture was then centrifuged at 1000 rpm for 10 minutes to pellet the cells. The supernatant was decanted and the cell pellet was then resuspended in freeze medium containing nutrient medium (1:1 combination of Dulbecco's modified Eagle's and Ham's F-12 medium with Glutamax-I (DMEM/F12) (Gibco, USA)), 8% FCS, 1 X antibiotics (penicillin, streptomycin and amphotericin B) (Lonza, USA) and 5% DMSO (Sigma, USA), and frozen overnight at -70°C, followed by storage in liquid nitrogen ($LN_2$).

The frozen cells were removed from the liquid nitrogen, hand warmed and resuspended in pre-warmed (37°C) DMEM/F12 with 10% FCS and 1X antibiotics. The mixture was centrifuged at 1000 rpm for 10 minutes to pellet the cells. The supernatant was decanted and fresh medium added. The reconstituted cells were then seeded into 25 $cm^2$ cell culture flasks and incubated at 37°C with 5% $CO_2$, until they grew to confluency. Cells were monitored for contaminants, before further use.

The virulent LSDV Warmbaths field isolate (LSDV WB) (GenBank Accession No. AF409137.1), from the Warmbaths (Bela-Bela) region in the Limpopo Province of South Africa, was used as parental virus in the construction of the recombinant virus and was also used as the challenge virus in the cattle trial. FBT and FCE cells were used for construction and selection of the recombinants and were grown in DMEM/F12 culture medium containing 8% FCS with 1X antibiotics.

The Onderstepoort Biological Products (OBP) lumpy skin disease vaccine (LSDV Neethling strain, GenBank Accession no: AF409138.1) was used in the cattle trial as the positive control virus. The vaccine was developed from a virulent field isolate over 50 years ago by 61 serial passages in lamb kidney cell monolayers followed by 20 passages in the chorio-allantoic membranes (CAMs) of embryonated hen's eggs, and another three passages in lamb kidney monolayers, ten times in Madine-Darby bovine kidney (MDBK) cells and finally five times in foetal bovine testes (FBT) cells (van Rooyen et al. 1969).

The parental virus used in the generation of the recombinant virus, and later the pure recombinant vaccine construct, were titrated on mycoplasma-free MDBK cells (prepared by D. Wallace). MDBK cells were seeded in a 12-well cell culture plate and incubated for approximately six hours at 37°C with 5% $CO_2$. 100 μl of the virus stock for titration was sonicated for 3 minutes in a sonicator (Sonorex® TK52, Bandelin, Germany) and added to 900μl of cell culture medium with 1X antibiotics, but no foetal calf serum. The medium in the wells was replaced with RPMI 1640 medium (Gibco, USA) containing 2% FCS and 1X antibiotics. A ten-fold serial dilution of the virus stock was then performed and 100μl per dilution added in quadruplicate to the wells. The cells were monitored for viral-induced cytopathogenic effects (CPE) after 3 to 4 days. The titre of the virus stock (N) was then calculated by counting plaques in the wells at the highest dilution in which plaques were observed and using the following formula:

$$N = \frac{\text{Average number of plaques in the 4 wells}}{d \times V} \quad \text{pfu/ml}$$

d = dilution factor
V = volume of diluted virus added to the wells (ml)
pfu/ml = plaque forming units per millilitre

**Recombinant virus generation and selection (rLSDV WB005KO)**

Primary FBT cells were seeded in 12-well cell culture plates (Nunclon) at 1 x $10^5$ cells per well in 1 ml of cell culture medium. Once they attained 80% confluency they were infected with the LSDV WB (Genbank Accession No. AF409137.1) parental virus at a multiplicity of infection (MOI) of 0.1. Transfection complexes were prepared using jetPEI® transfection reagent (Polyplus transfection) according to the manufacturer's instructions. 1 μg of highly pure pTW005 insertion vector DNA (purified using the Qiagen® Midiprep kit) was diluted in 150 mM NaCl (made up to final volume of 50 μl) and 2 μl of jetPEI® reagent in 150 mM NaCl (made up to a final volume of 50 μl per well). The jetPEI® and DNA solutions were mixed, vortexed and incubated for 30 minutes at room temperature. One hour post-infection, 100 μl of the jetPEI® DNA mixture was added drop-wise to the cells in 1 ml of serum-containing medium per well. Plates were gently swirled and then returned to the incubator (37°C, 5% $CO_2$) overnight. The transfection mixture was removed and replaced with DMEM/F12, antibiotics and 8% FCS, and the cells were incubated for another 4 days, or until complete CPE was observed and easily visualised using an inverted fluorescence stage microscope under UV light examination (cells containing recombinant virus produced visible green light due to the expression of the EGFP selection marker protein).

Cells were then freeze-thawed three times and the solution cleared by centrifugation. A 10-fold serial dilution was then made of the supernatant and an aliquot of each dilution was placed onto fresh FBT cells pre-incubated for 24 h in the presence of MXH selection medium (10 μg/ml mycophenolic acid, 250 μg/ml xanthine, 15 μg/ml hypoxanthine, DMEM/F12, 2.5% FCS and 1X antibiotics). Cells were incubated under standard conditions (37°C, 5% $CO_2$), with replacement of the selection medium every 72 h until CPE became visible (usually 4-5 days post-inoculation). The freeze-thawing, serial dilutions and incubation under standard conditions were repeated once more.

After 4-5 days, once CPE exhibiting fluorescing virus appeared, the selection medium was removed from the cells and dead cells were pelleted at low speed (200 g, 5 min) in a bench-top centrifuge. The supernatant fluid was subjected to sonication at 35 kHz for 15 min in a water-bath sonicator (Sonorex® TK52, Bandelin, Germany) and then filtered through a 0.45 μm pore acetate filter (Millipore, USA). The filtrate was diluted to end-point using a 10-fold serial dilution, and placed onto fresh FBT cells under selection (as described above). The supernatant fluid from the well exhibiting foci at the highest dilution was again removed, separated from dead cells, sonicated, filtered, and titrated. For the third cycle the cells in the well exhibiting individual fluorescing foci at the highest dilution were washed in sterile PBS and individual fluorescing-foci were picked using a sterile 20 μl filtered pipette tip. These were then freeze-thawed as described, and half of the material collected from each focus was inoculated onto fresh cells under selection in 6-well or 12-well culture dishes (Nunclon), while the other half was stored at -20°C.

PCR was used at every step in the selection process to determine whether the recombinant virus stock was approaching homogeneity, free from wild-type/parental virus.

**PCR screening for homogeneity**

Two primers were used: LF005FWD (5'-ATA CAT CTT AAG CAGT TTT AAT CGC TCT AAA ATT C-3') (SEQ ID NO:3), which binds specifically to sequences just upstream of the putative IL-10-like gene and RT005REV (5'-ATC GAG CTC TAT CGT TTG TAA GAA ATT ATA ACG-3') (SEQ ID NO:6), which binds to sequences downstream of the putative Interleukin-10-like gene.

A confluent monolayer of FBT cells in a 6-well cell culture plate was infected with rLSDV WB005KO foci removed from storage at -20°C and thawed at room temperature at a low MOI. The plate was then incubated at 37°C, in 5% $CO_2$ for 48 h. The virus-infected area and foci were scraped with a micropipette tip while aspirating 10 μl of medium and cells. This was dispensed into a new microfuge tube and 10 μl of detergent solution (2X PCR Buffer with 0.9% NP40 and 0.9% Tween-20®) or (2X PCR Buffer with 2% Triton X-100) and 1.2 μl of proteinase K (5 mg/ml) was added. This was incubated for 30 minutes to 1 hour at 37°C or 30 minutes at 45°C. The proteinase K was then heat inactivated for 10 minutes at 94°C and then centrifuged at high speed at (14000 g) for 10 seconds to remove cell debris. Ten μl of the supernatant was then used in a 100 μl PCR reaction.

PCR was performed on the supernatant using TaKaRa Ex Taq™ DNA Polymerase (Takara, Japan) according to manufacturer's guidelines. The PCR was carried out with an initial denaturing step at 98°C for 5 minutes, followed by 35 cycles at 98°C for 1 minute, 58°C for 30 seconds, 72°C for 5 minutes and a final elongation step of 72°C for 10 minutes. The samples were then subjected to agarose gel electrophoresis using a 1.2% agarose gel.

Once foci were obtained which showed the absence of the wild-type virus, selection pressure was lifted and the rLSDV WB005KO virus was passaged on FBT cells containing DMEM/F12 with 8% FCS and antibiotics only. Cells were passaged a number of times and were checked for CPE. PCR was used once more to confirm the absence of wild-type virus.

**Recombination and screening of the recombinant rLSDV WB005KO virus for removal of the gpt antibiotic resistance marker gene.**

Removal of the selection pressure had a secondary purpose - to allow a second round of homologous recombination for the removal of the gpt gene. The process was repeated as described above. PCR was used at every step in the process to determine whether the gpt gene had been removed from the recombinant virus. Two primers: Eco-gpt-F (5'-CAG GCT GGG ACA CTT CAC-3') (SEQ ID NO:7) and Eco-gpt-R (5'-GAT TAG CGA CCG GAG ATT G-3') (SEQ ID NO:8), which bind to the 5' and 3' regions of the gpt gene, respectively, were used to confirm removal of the gpt selection marker gene.

In brief, a confluent monolayer of FBT cells in a 6-well tissue culture plate was infected with rLSDV WB005KO at a low MOI. The plate was incubated at 37°C, 5% $CO_2$, for 48 h. The virus-infected cells were scraped loose using a sterile micropipette tip and recovered in 10 μl of medium, using gentle aspiration. The virus-cell mixture was dispensed into a microfuge tube and 10 μl of detergent solution (2X PCR Buffer containing 0.9% NP40 and 0.9% Tween®) and 1.2 μl of proteinase K (5 mg/ml) were added. This was incubated for one hour at 37°C. The proteinase K (Fermentas) was then heat inactivated for 10 minutes at 94°C, followed by centrifugation at high speed at 14000 g for 10 seconds to remove cell debris.

PCR was performed on 10 μl of the recovered supernatant using TaKaRa Ex Taq™ DNA Polymerase (Takara, Japan) according to the manufacturer's guidelines in a 100 μl reaction volume. The PCR was carried out using an initial denaturation step at 98°C for 5 minutes, followed by 35 cycles at 98°C for 1 minute, 58°C for 30 seconds, 72°C for 5 minutes and a final elongation step at 72°C for 10 minutes. The sample was then subjected to agarose gel electrophoresis using a 1.2% agarose gel for analysis.

The final homogeneous LSDV WB005 KO construct, containing the EGFP marker gene without the gpt gene, was grown to high titres in FBT cells and was used to evaluate its potential as a vaccine in protecting cattle against LSD challenge, goats against goat pox challenge and sheep against sheep pox challenge.

## EXAMPLE 2

### Vaccination and experimental challenge in cattle

The example was aimed at evaluating the recombinant LSDV (putative) IL-10-like gene knockout (LSDV WB005KO) construct for use as a vaccine, to protect cattle against LSD challenge. The OBP LSDV Neethling vaccine strain, which is produced by OBP (GenBank Accession no: AF409138.1) and from here on referred to as "LSDV OBP Vac", was used as a positive control virus. The challenge virus was the virulent LSDV Warmbaths field isolate (LSDV WB) (GenBank Accession no. AF409137.1).

### Inoculation of the calves with LSDV OBP Vac and LSDV WB005KO

A total of thirty 6-month old Holstein calves were pre-screened for LSDV antibodies by serum neutralisation testing. All the animals tested negative. Twenty calves were selected, purchased, delivered, and kept in an insect free-facility. Within the first week of arrival, serum samples were obtained from the calves and retested for the presence of LSDV antibodies. The calves were allowed to acclimatise for three weeks and rectal temperatures were taken daily.

Fifteen calves were divided into three groups of five each and were vaccinated subcutaneously (SC) in the neck as indicated in Table 1.

Table 1: Animals vaccinated with different inocula

| Group | Inoculum | Vaccination Titre | Volume of inoculum |
|---|---|---|---|
| 1* | (DMEM/F12 Antibiotics) | N/A | 1 ml |
| 2 | LSDV OBP Vac | 2.2 x $10^3$ pfu** | 2 ml |
| 3 | LSDV WB005KO | 1 x $10^7$ pfu | 2 ml |
| * - Group 1: Negative control<br>** - pfu = plaque forming units | | | |

Rectal temperatures were measured daily following vaccination and the calves were monitored for clinical signs of illness according to a score sheet (reaction at site of inoculation, loss of appetite, lesions, heat, pain, oedema and lymphadenopathy). Both humoral and cell-mediated immune responses, as well as viraemia, were measured following vaccination.

### Responses elicited by the LSDV WB005KO construct following vaccination

Table 2 indicates the parameters that were measured and the results that were obtained following vaccination. Briefly, three calves vaccinated with LSDV WB005KO developed fever as compared to the negative control and OBP vaccine groups. No clinical symptoms were noted in the two control groups. Post-vaccinal reactions, believed to be LSDV-specific, were noted in the LSDV WB005KO vaccinated group (Figure 3). This group also showed detectable antibody responses by day 10 post-vaccination as compared to the two control groups which showed no detectable antibody responses (Figure 4). Protection against LSDV infection is considered to be mainly cell-mediated, thus it is significant that animals in this group showed a better cellular immune response as compared to the OBP vaccine and negative control groups, following vaccination (Figure 5).

Table 2: Results following vaccination

| Measured Parameters | Negative control | OBP Vaccine | LSDV WB005 KO |
|---|---|---|---|
| Temperature | No changes | No changes | 3 calves reacted above 39°C, peaking at 40°C in one animal. |
| Clinical signs | No clinical signs | No clinical signs | Large localised reaction at vaccination site in 3 of the calves. 2 calves showed lesions around the cranial and shoulder area. 1 calf died of rumen acidosis (Day 18, No: 3135, unrelated to vaccination). |

(continued)

| Measured Parameters | Negative control | OBP Vaccine | LSDV WB005 KO |
|---|---|---|---|
| Serology (Serum Neutralisation Test) | No detectable levels | No detectable levels | All calves showed neutralising antibodies.<br>4 of the calves by Day 10. |
| Virus isolation - Blood | Negative | Unable to isolate virus after 3 passages | Unable to isolate virus after 3 passages. |
| Virus isolation - biopsy | None taken | None taken (No lesion at site of inoculation) | Sample taken from 2 calves at site of inoculation.<br>Both tested positive for LSDV DNA by PCR. |
| Viraemia (PCR) | Negative | Unable to detect LSDV DNA by PCR | Unable to detect LSDV DNA by PCR |

**Responses elicited by the LSDV WB005KO construct following challenge with the virulent LSDV WB isolate**

Twenty-eight days after vaccination the calves were challenged intradermally (ID) with the virulent LSDV Warmbaths field isolate (Genbank Accession No. AF409137.1) by injecting 500 μl (5 x $10^6$ pfu) on either side of the neck.

Table 3 indicates the parameters that were measured and the results that were obtained following challenge. One of the calves in the LSDV WB005KO group (No. 3135) died, due to circumstances unrelated to the trial on day 18. Rectal temperatures of the calves were also measured daily following challenge and the calves were monitored daily for clinical signs of illness according to the score sheet. Humoral and cell-mediated immunity, as well as viraemia, was measured following challenge. Animals in the control groups developed fevers as noted, while the LSDV WB005KO group showed no febrile response. Animals in the two control groups developed crust-like lesions at the sites of inoculation (Figure 6), while no lesions were observed in the LSDV WB005KO group. Neutralising antibodies were detected in all of the animals post-challenge with exceedingly high titres recorded in sera from two of the animals in the KO group (Figure 7). The cellular response measured was higher in the negative control and OBP vaccine groups on day 7 post-challenge (Figure 9).

Table 3: Results following challenge

| Measured Parameters | Negative control | OBP Vaccine | LSDV WB005 KO |
|---|---|---|---|
| Temperature | 2 calves reacted, above 39°C peaking at 40.5°C | 5 calves reacted, above 39°C peaking at 41°C | No changes |
| Clinical signs | 5 calves developed lesions that became crusts | 5 calves developed lesions that became crusts | No clinical signs |
| Serology (Serum Neutralisation Test) | All calves showed neutralising antibodies by Day 17 | All calves showed neutralising antibodies by Day 17 | All 4 calves showed neutralising antibodies. Maximum titre as measured in 2 calves |
| Virus isolation - Blood | Negative | Negative | Negative |
| Virus isolation - biopsy | Taken post-mortem.<br>Negative by PCR. | Taken post-mortem.<br>Negative by PCR. | None taken.<br>No lesion at site of inoculation |
| Viraemia (PCR) | Unable to detect LSDV DNA by PCR | Unable to detect LSDV DNA by PCR | Unable to detect LSDV DNA by PCR |

**Humoural immunity - Serum neutralisation testing**

Serum neutralisation testing was performed according to Beard et al., 2010. All procedures were carried out under sterile conditions. Briefly, a 1:5 dilution of the test serum was made in $PBS^+$ buffer and inactivated in a waterbath for 30 min at 56°C. A series of six two-fold dilutions in Minimum Essential Media (MEM) culture medium (Gibco, UK) containing 5% FCS was made in 96-well microtitre plates from the 1:5 dilution of the test serum in 100 μl volumes. The

stock virus (LSDV WB005KO) was diluted in MEM culture medium containing 5% FCS foetal calf serum to obtain a concentration of 100 $TCID_{50}$/ml. A series of four, ten-fold dilutions was then made from this viral antigen stock, to be used as the virus control. Fifty μl was added to all wells containing the diluted test serum. A virus control was prepared covering three rows and six columns as follows:

a. 100 μl MEM containing 5% FCS was added to all the wells.
b. 100 μl of the 100 $TCID_{50}$/ml virus stock was added to the first two columns.
c. The remaining four columns received 100 μl aliquots of the virus dilutions, starting with the highest dilution in column 3.

The plates were incubated for one hour at 37°C (± 2°C) in a humid atmosphere containing 5% $CO_2$. During this period FBT cells (uninfected) were trypsinised, harvested and counted. Cells were then diluted to 4.8 x $10^5$ cells/ml and 80 μl was added to each well in the titration plate, with at least 2 rows serving as the cell control, thus receiving only cells and 200 μl of MEM with 5% FCS.

The plates were then incubated as above, until the cells in the wells containing the back-titrated stock virus showed 50% CPE at the $10^{-2}$ dilution. The presence of neutralising antibodies in the test sera inhibits the production of CPE. Thus an end point antibody titre was calculated as the dilution at which 50% of the cells exhibited CPE.

**Cell-mediated immunity - Lymphocyte proliferation assay**

Lymphocyte proliferation assays were carried out in triplicate in 96-well plates as described by Van Kleef et al., 2000. Briefly, PBMCs (2 x $10^5$ PBMCs/well) recovered from blood from the trial cattle were stimulated with different antigens in triplicate in a total volume of 100 μl in complete RPMI-1640 medium (Gibco, UK). Antigens used for the stimulation were the LSDV WB virus (that was added at 1 x $10^5$ pfu/well), negative antigen (unstimulated PBMCs) and ConA (5.0 μg/ml, Sigma). The cells were incubated at 37°C in a humidified atmosphere with 5% $CO_2$ for 3-5 days. The cells were then labelled with 1 μCi/well of [$^3$H] thymidine and incubated for an additional 18 h as above. Cells were harvested and the radioactivity was measured using a Trilux® 1450 Microbeta® scintillation counter (Wallac). The results were expressed as stimulation index (SI) values, which are defined as the mean counts per minute of the test antigen divided by the mean counts per minute (cpm) of the unstimulated PBMCs. A response was considered positive if the result was two times higher than the SI of the negative control with a significant p-value (p-value ≤ 0.01, as determined by Student's t-test).

**Conclusion**

The LSDV IL-10-like gene KO construct induced detectable antibody responses at 10 days post-vaccination compared to none in the control groups and also induced a better cellular response as compared to the current LSDV OP vaccine. Animals in the LSDV IL-10 like gene KO group also had a rise in temperature following vaccination and a response at the primary site of inoculation. The response at the primary site of inoculation may be due to the high titre of virus that was administered. Following challenge only some of the animals in the control groups developed a fever while all developed a crust like lesion at the site of inoculation as compared to the LSDV IL-10-like gene KO group which showed none. All the animals in the control groups showed detectable antibody responses by day 16 post challenge.

By contrast, good humoral and cellular immune responses were measured in most of the animals inoculated with the LSDV WB005 KO construct at a high dose. In addition, most of the animals did not develop severe clinical signs following both vaccination and challenge. This suggests the construct has good potential as a new LSD vaccine - pending further safety and efficacy trials.

**EXAMPLE 3**

**Vaccination and experimental challenge in sheep and goats**

Twelve Boer cross goats and twelve Rideau Arcott sheep (all 6-months old) were obtained from local farms, and were housed in separate Biosafety Level 3 animal cubicles at the National Centre for Foreign Animal Disease (Winnipeg, Canada). The sheep were randomly divided into two groups of six each for use as vaccinated and non-vaccinated controls, as were the goats. All animals were fed a complete balanced diet and water *ad libitum.* Animal experimentation was conducted under the approval of the Canadian Science Centre for Human and Animal Health Animal Care Committee, which follows the guidelines of the Canadian Council on Animal Care. Both sheep and goats were screened and found negative for capripoxvirus by real-time PCR and serology prior to vaccination.

The recombinant virus construct, LSDV WB005KO, was diluted in DMEM to a concentration of 3 x $10^3$ $TCID_{50}$/ml.

Six sheep and six goats were vaccinated with 0.1 ml injected intradermally. Following vaccination, blood, sera, oral and nasal swabs were collected at 4, 6, 8, 10, 12, 14 and 21 days post inoculation (dpi). Unvaccinated control sheep and goats were injected with DMEM only. After observation of the animals for 21 days, both the vaccinated and unvaccinated groups were housed together prior to challenge using virulent capripoxvirus strains. Viral challenge was performed in both vaccinated and non-vaccinated animals. Sheep were challenged using a 0.1 ml intradermal injection of the Nigerian isolate of sheep pox virus ($10^5$ $TCID_{50}$/ml) (Bowden et al., 2008) and goats using a 0.1 ml intradermal injection of the Yemen strain of goat pox virus ($10^5$ $TCID_{50}$/ml) (Babiuk et al., 2009). All animals were observed daily and clinical signs recorded throughout the study. Rectal temperatures were measured daily for up to 21 dpi. Blood and sera were collected from sheep and goats at 4, 6, 8, 10, 12, 14 and 21 dpi.

## Capripoxvirus antibody detection ELISA

Antibodies generated by vaccination or viral challenge were quantified using an ELISA protocol and two recombinant capripox virion core proteins (ORF 095 and ORF 103) expressed in *E. coli* as previously described (Bowden et al., 2009). Briefly, ninety-six well ELISA plates (Nunc, USA) were coated with a combination of the bacterially-expressed recombinant capripoxvirus proteins, ORF 095 and ORF 103, with 30 ng of each protein diluted in 100 μl carbonate buffer (pH 9.6) and incubated overnight at 4°C. The plates were then incubated with blocking solution (5% skim milk in PBS/0.05% Tween®) for 1 hour at 37°C. Serially-diluted sheep and goat sera (starting at a 1:100 dilution) were then added, incubated for 1 hour at 37°C, washed 3 times, and further incubated for 1 hour at 37°C with a 1:32 000 dilution of peroxidase-conjugated protein G (Sigma, USA). The plates were then washed 3 times, developed using Blu Phos™ phosphatase substrate (KPL, USA) and absorbance measured at a wavelength of 650 nm. Endpoint titres were determined using the average optical density plus two standard deviations from negative sheep sera as the cut-off value.

## Virus neutralisation test (VNT)

Virus neutralising antibodies that were generated either by vaccination and/or viral challenge were quantified using VNT. Serial dilutions (from 1:10 to 1:10,240) of sheep and goat sera, at various time points following vaccination and challenge, were evaluated. The Kenyan vaccine strain of sheep and goat pox virus (KS-1) (100 $TCID_{50}$, in DMEM) was mixed with sheep and goat sera (in duplicate) to a volume of 200μl, and incubated for 1 hour at 37°C. Vero cells incubated in 96-well plates were infected with the 200μl mixture of virus and serum (or media, as negative control). The cells were then incubated for 6-7 days, with daily examination for CPE. A virus neutralising titre was determined to be the highest dilution that displayed reduced CPE when compared to a previously characterized negative control.

## Capripoxvirus real-time PCR

To quantify the presence of viral DNA in blood, oral and nasal swabs were tested from sheep and goats following vaccination, and in blood following viral challenge. A quantitative real-time PCR Taqman® assay was performed using the protocol previously described (Bowden et al., 2008). This assay detects an 89 bp region of capripoxvirus ORF 074. Blood and swabs were collected at 0, 4, 6, 8, 10, 12, 14 and 21 days post-vaccination. Blood was collected at 4, 6, 8, 10, 12, 14 and 21 days post-challenge.

## ELISA-based detection of sheep and goat interferon-gamma (IFN-γ)

Blood was collected from both sheep and goats 21 days post-vaccination in vacutainer tubes (Becton Dickinson, USA). Purified leukocytes were analysed for their ability to secrete IFN-γ in the presence of capripoxvirus antigen using a method previously described (Caro et al., 1998), with modifications. A stock of KS-1 capripoxvirus ($1 \times 10^5$ $TCID_{50}$) was heat-inactivated, and a 1/100 dilution of the virus was incubated with $2 \times 10^5$ test-sample leukocytes in a 96-well plate for 48 hours in a total volume of 200 μl. Supernatants were then collected and quantified using a bovine quantitative IFN-γ kit (AbD Serotec, USA), which can be used to measure sheep and goat IFN-γ based on the ability of selected bovine monoclonal antibodies to cross-react with both sheep and goat IFN-γ. Secreted IFN-γ levels were measured using a quantitative ELISA, as previously described (Tourais-Esteves et al., 2008). Total IFN-γ concentrations were determined by normalisation with a standard provided by the manufacturer.

## Statistics

Statistical analyses were performed using a t-test in Excel (Microsoft, USA) to determine differences between sheep and goats for viral DNA loads, serology and IFN-γ secretion.

## Safety of the LSDV WB 005 KO in sheep and goats

Sheep and goats were housed in high-containment animal care facilities, with the vaccinated and control groups in separate cubicles, allowing them to acclimate to their surroundings for at least two weeks prior to vaccination. During that time their body temperatures were recorded daily, and they were monitored for overall health and wellbeing. All animals were healthy and free of disease, and had basal rectal temperatures ranging from 38.5-40.1°C (goats) and 39.0-39.8°C (sheep). Following vaccination, the appearance of injection site reactions ranged from invisible to a single, raised, reddened nodule of approximately 1 cm in diameter which disappeared within 2 weeks. To evaluate the replication of the LSDV WB 005 KO construct in vaccinated animals, blood, oral and nasal swabs were evaluated by real-time PCR for the presence of capripoxvirus DNA. No vaccinated sheep or goats had detectable viral DNA in their blood, or from oral or nasal swabs, at any time points following vaccination, indicating that the vaccine did not replicate beyond the injection sites.

## Fever and disease progression in sheep and goats

Twenty days following vaccination, both vaccinated and unvaccinated animals were housed together prior to challenge with virulent capripoxviruses. Following challenge, sheep and goats were monitored for fever. Elevated rectal temperatures were observed in non-vaccinated controls starting at 5 days post-challenge (dpc) for sheep (Figure 9A) and at 6 dpc for goats (Figure 9B). Elevated temperatures in these animals were observed until full recovery from clinical disease.

Clinical signs of capripoxvirus infection were observed in non-vaccinated sheep and goats, whereas all six vaccinated sheep (Figure 10B) and all six vaccinated goats (Figure 10C) did not develop clinical disease. All six non-vaccinated sheep developed numerous pox skin lesions (Figure 10F), most easily observed on the skin in areas without wool, conjunctivitis (Figure 10A) and mucopurulent nasal discharge. All non-vaccinated goats also displayed numerous pox skin lesions (Figure 10D), conjunctivitis, mucopurulent nasal discharge (Figure 10E), and in addition, severe lethargy.

## Replication of pathogenic capripoxvirus in sheep and goats

In order to measure viral replication in both vaccinated and unvaccinated animals, quantitative PCR was performed using whole blood from both sheep and goats. All vaccinated animals did not have any measurable degree of viraemia (Figure 11). In contrast, unvaccinated animals developed detectable capripoxvirus DNA using real-time PCR, with viral DNA levels peaking at 8 dpc in sheep and 12 dpc in goats. By day 21, none of the animals used in this study had detectable levels of viraemia.

## Serology following vaccination and challenge

Sera were evaluated at multiple time points in sheep and goats following vaccination and capripoxvirus challenge. Sera from all time points were analysed for IgG antibodies specific for recombinantly-expressed capripox virion core proteins by ELISA. Neutralising antibodies were also measured by assessing the inhibition of KS-1 capripoxvirus incubated in the presence of serum samples to infect OA3.T cells *in vitro.*

Following vaccination with the LSDV WB 005 KO vaccine construct, sheep developed low levels of capripoxvirus-specific IgG antibodies starting at 4 days post-vaccination (dpv), as measured using a recombinant antigen ELISA (Figure 12). In contrast, goats did not develop any detectable levels of capripoxvirus-specific IgG antibodies post-vaccination. Following capripoxvirus challenge, titres in vaccinated sheep did not significantly increase, whereas unvaccinated sheep developed significant levels of capripoxvirus-specific IgG antibodies. Vaccinated sheep generated statistically relevant lower titres (P=0.017) than the unvaccinated sheep following capripoxvirus challenge. In goats, following capripoxvirus challenge, both vaccinated and unvaccinated goats developed capripoxvirus-specific IgG antibodies. Vaccinated goats had significantly lower antibody titres compared to non-vaccinated goats (P=0.001).

Following vaccination, sheep developed significant low levels of detectable capripoxvirus neutralising antibodies as measured using VNT, whereas goats did not until 21 dpv (Figure 13). Following capripoxvirus challenge, both vaccinated and unvaccinated sheep and goats elicited a significant increase in VNT titres. Unvaccinated control sheep and goats had higher VNT titres compared with vaccinated sheep (P=0.053) and goats (P=0.041).

## Quantification of goat IFN-γ following vaccination with LSDV WB005KO

PBMCs were collected from all goats (vaccinated and unvaccinated) at 14 and 21 dpv and were analysed for their ability to secrete IFN-γ in the presence of capripoxvirus antigen. The PBMCs were incubated in the presence of heat-inactivated capripoxvirus strain KS-1 for 2 days, and then assessed for IFN-γ secretion. The average levels of

secreted IFN-$\gamma$ from vaccinated goat PBMCs was greater than 800 pg/ml at 14 dpv and 2400 pg/ml at 21 dpv (Figure 14). No significant levels of IFN-$\gamma$ was detected in either the unvaccinated control PBMCs, or in PBMCs stimulated without virus, indicating the responses observed in PBMCs from vaccinated goats were capripoxvirus-specific antigen recall responses.

## Conclusion

The efficacy of the LSDV WB005KO construct, as a vaccine, was evaluated in both sheep and goats, as it has been previously shown that immunity against LSDV can confer protection against both sheep pox and goat pox (OIE, 2010; Capstick, 1961; OIE, 2009). It was found that vaccination of both groups of animals with the vaccine construct did not elicit any clinical disease, and skin reactions at the injection sites were minimal.

Furthermore, sera collected from vaccinated sheep and goats prior to capripoxvirus challenge were found to have significant capripoxvirus neutralising antibody levels, indicating that the vaccine induced capripoxvirus-specific antibody production. Interestingly, both neutralising and specific antibodies to capripoxvirus core proteins increased following capripoxvirus challenge in sheep and goats. However, levels of capripoxvirus antibodies from vaccinated sheep and goats were lower following challenge compared to unvaccinated animals. One possible reason for this is that the vaccine induced a strong cellular immunity in vaccinated animals, leading to decreased virus replication, compared with unvaccinated controls. This decrease in virus replication was possibly responsible for differences in the amount of antigen available to stimulate B-cells, allowing the unvaccinated control animals to generate higher levels of capripoxvirus-specific antibodies.

In addition to the antibody responses elicited by the LSDV WB005KO vaccine in sheep and goats, it is known that cell-mediated immunity is also elicited by capripoxvirus infection as killed capripoxvirus vaccines are not effective in controlling the disease. Cell-mediated immunity was confirmed by analysis of PBMCs isolated from vaccinated and unvaccinated goats, with significant IFN-$\gamma$ production observed 14 and 21 days post-vaccination.

The efficacy of the LSDV WB005KO construct was evaluated in both sheep and goats by viral challenge using virulent capripoxvirus isolates, which have been demonstrated previously to cause clinical disease in both field and experimental settings. There was no disease progression in vaccinated sheep and goats, compared to unvaccinated controls which displayed clinical capripoxvirus disease, including viral DNA detection in blood by real-time PCR, fever, skin lesions, and nasal discharge. The presence of viral DNA in blood from all vaccinated sheep and goats was below the limit of detection, suggesting that sterile immunity may have been achieved. These results demonstrate the effectiveness of the LSDV WB005KO vaccine construct used at doses that would be practical for mass vaccination in the field. The efficacy of this vaccine, as well as its design, allows for the possibility of its widespread use following field trials and licensing in either: a) its current form, or; b) as a potential recombinant vaccine vector, expressing antigens from other pathogens.

## REFERENCES

Babiuk, S., et al. (2009) J. Gen. Virol. 90: 105-114.
Babiuk, S., et al (2008) Transbound Emerg. Dis. 55: 263-272.
Beard P.M., et al. (2010) Veterinary Microbiology 142: 427-431.
Bhanuprakash, V., et al. (2006) Comp. Immunol. Microbiol. Infect. Dis. 29: 27-60.
Bowden, T.R., et al. (2009) J. Virol. Methods 161: 19-29.
Bowden, T.R., et al. (2008) Virology 371, 380-393.
Capstick, P.B. (1961) Veterinary Department Annual Report. Government of Kenya. , 45-47.
Caro, M.R., et al. (1998) Res. Vet. Sci. 65, 145-148.
Davies, F.G. and Mbugwa, G., (1985) J. Comp. Pathol. 95, 565-572.
Esposito and Knight. (1985) Virology. 143(1), 230-51.
Hunter and Wallace. (2001) Journal of the South African Vet Ass, 72(2), 68-71.
Kara, et al. (2003) Arch Virol. 148(7), 1335-56.
Kitching, R.P. (2003) Dev. Biol. (Basel) 114, 161-167.
OIE, 2010. Sheep And Goat Pox; OIE Terrestrial Manual, pp. 2.7.14.
OIE, 2009. Sheep and Goat Pox--World Animal Health/OIE bulletin.
Tourais-Esteves, I., et al. (2008) Dev. Comp. Immunol. 32, 1231-1241.
Van Kleef M., et al. (2000) Infection and Immunity. 68(2): 603-614.
Van Rooyen, P.J., et al. (1969) Onderstepoort J. of Vet. Res. 36: 165-174.
Weiss. (1968) Virology Monographs. 3, 112-282.

## Claims

1. A lumpy skin disease virus (LSDV) interleukin-10-like gene knock-out mutant, wherein the interleukin-10-like gene has been inactivated by deletion from the viral genome.

2. A pharmaceutical composition, comprising:

i. a LSDV interleukin-10-like gene knock-out mutant, wherein the interleukin-10-like gene has been deleted from the viral genome; and
ii. a pharmaceutically acceptable diluent or excipient.

**3.** The pharmaceutical composition of claim 2, which further includes an adjuvant.

**4.** The pharmaceutical composition of claim 3, wherein the pharmaceutical composition is capable of eliciting a protective immune response in an animal against a disease caused by a virus from the viral genus *Capripoxvirinae.*

**5.** The pharmaceutical composition of claim 4, wherein the disease is selected from lumpy skin disease, sheep pox and goat pox.

**6.** The pharmaceutical composition of claim 4 or 5, wherein the animal is a ruminant.

**7.** The pharmaceutical composition of claim 6, wherein the ruminant is selected from the group consisting of cattle, sheep, goats and wild ungulates.

**8.** The pharmaceutical composition of any one of claims 4 to 7, which is formulated for intramuscular, intradermal, intravenous or subcutaneous administration.

**9.** The pharmaceutical composition of any one of claims 4 to 8, wherein the composition is formulated for administration to the animal in a single dose.

**10.** The pharmaceutical composition of any one of claims 4 to 8, wherein the composition is formulated for administration to the animal in two or more doses.

**11.** The LSDV knock-out mutant of claim 1 for use in a method of preventing a disease in an animal, wherein the disease is caused by a virus from the viral genus *Capripoxvirinae,* and optionally, wherein the disease is selected from lumpy skin disease, sheep pox and goat pox.

**12.** The LSDV knock-out mutant for use according to claim 11, wherein the animal is a ruminant, and optionally, wherein the ruminant is selected from the group consisting of cattle, sheep, goats and wild ungulates.

## Patentansprüche

**1.** Lumpy-Skin-Disease-Virus (LSDV)-Interleukin-10-artiges-Gen-Knockout-Mutante, wobei das Interleukin-10-artige-Gen durch Deletion aus dem viralen Genom inaktiviert wurde.

**2.** Pharmazeutische Zusammensetzung, umfassend:

i. eine LSDV-Interleukin-10-artiges-Gen-Knock-out-Mutante, wobei das Interleukin-10-artige-Gen aus dem viralen Genom deletiert wurde; und
ii. einen pharmazeutisch annehmbaren Verdünner oder Exzipienten.

**3.** Pharmazeutische Zusammensetzung nach Anspruch 2, die ferner ein Adjuvans einschließt.

**4.** Pharmazeutische Zusammensetzung nach Anspruch 3, wobei die pharmazeutische Zusammensetzung zum Hervorrufen einer schützenden Immunantwort in einem Tier gegen eine Krankheit in der Lage ist, die durch ein Virus aus der viralen Gattung *Capripoxvirinae* verursacht wird.

**5.** Pharmazeutische Zusammensetzung nach Anspruch 4, wobei die Krankheit ausgewählt ist aus Lumpy-Skin-Krankheit, Schafpocken und Ziegenpocken.

**6.** Pharmazeutische Zusammensetzung nach Anspruch 4 oder 5, wobei das Tier ein Wiederkäuer ist.

**7.** Pharmazeutische Zusammensetzung nach Anspruch 6, wobei der Wiederkäuer aus der Gruppe bestehend aus Rindern, Schafen, Ziegen und wilden Huftieren ausgewählt ist.

**8.** Pharmazeutische Zusammensetzung nach einem der Ansprüche 4 bis 7, die zur intramuskulären, intradermalen, intravenösen oder subkutanen Verabreichung formuliert ist.

**9.** Pharmazeutische Zusammensetzung nach einem der Ansprüche 4 bis 8, wobei die Zusammensetzung zur Verabreichung an das Tier in einer Einzeldosis formuliert ist.

**10.** Pharmazeutische Zusammensetzung nach einem der Ansprüche 4 bis 8, wobei die Zusammensetzung zur Verabreichung an das Tier in zwei oder mehr Dosen formuliert ist.

**11.** LSDV-Knockout-Mutante nach Anspruch 1 zur Verwendung in einem Verfahren zur Verhinderung einer Krankheit bei einem Tier, wobei die Krankheit durch ein Virus aus der viralen Gattung *Capripoxvirinae* verursacht wird und wobei die Krankheit gegebenenfalls ausgewählt ist aus Lumpy-Skin-Krankheit, Schafpocken und Ziegenpocken.

**12.** LSDV-Knockout-Mutante zur Verwendung gemäß Anspruch 11, wobei das Tier ein Wiederkäuer ist und wobei der Wiederkäuer gegebenenfalls ausgewählt ist aus der Gruppe bestehend aus Rindern, Schafen, Ziegen und wilden Huftieren.

**Revendications**

**1.** Mutant inactivé du gène similaire à l'interleukine 10 du virus de la dermatose nodulaire (LSDV), dans lequel le gène similaire à l'interleukine 10 a été inactivé par délétion du génome viral.

**2.** Composition pharmaceutique, comprenant :

i. un mutant inactivé de gène similaire à l'interleukine 10 de LSDV, dans laquelle le gène similaire à l'interleukine 10 a été délété du génome viral ; et
ii. un diluant ou excipient pharmaceutiquement acceptable.

**3.** Composition pharmaceutique selon la revendication 2, qui comprend en outre un adjuvant.

**4.** Composition pharmaceutique selon la revendication 3, la composition pharmaceutique pouvant induire une réponse immunitaire protectrice chez un animal contre une maladie causée par un virus du genre viral *Capripoxvirinae.*

**5.** Composition pharmaceutique selon la revendication 4, la maladie étant choisie parmi la dermatose nodulaire, la variole ovine et la variole caprine.

**6.** Composition pharmaceutique selon la revendication 4 ou 5, l'animal étant un ruminant.

**7.** Composition pharmaceutique selon la revendication 6, le ruminant étant choisi dans le groupe constitué de bovins, moutons, chèvres et ongulés sauvages.

**8.** Composition pharmaceutique selon l'une quelconque des revendications 4 à 7, qui est formulée pour administration intramusculaire, intradermique, intraveineuse ou sous-cutanée.

**9.** Composition pharmaceutique selon l'une quelconque des revendications 4 à 8, la composition étant formulée pour administration à l'animal dans une dose unique.

**10.** Composition pharmaceutique selon l'une quelconque des revendications 4 à 8, la composition étant formulée pour administration à l'animal dans deux doses ou plus.

**11.** Mutant inactivé de LSDV selon la revendication 1, pour utilisation dans un procédé de prévention d'une maladie chez un animal, la maladie étant causée par un virus du genre viral *Capripoxvirinae,* et la maladie étant facultativement choisie parmi la dermatose nodulaire, la variole ovine et la variole caprine.

**12.** Mutant inactivé de LSDV pour utilisation selon la revendication 11, l'animal étant un ruminant, et le ruminant étant facultativement choisi dans le groupe constitué de bovins, moutons, chèvres et ongulés sauvages.

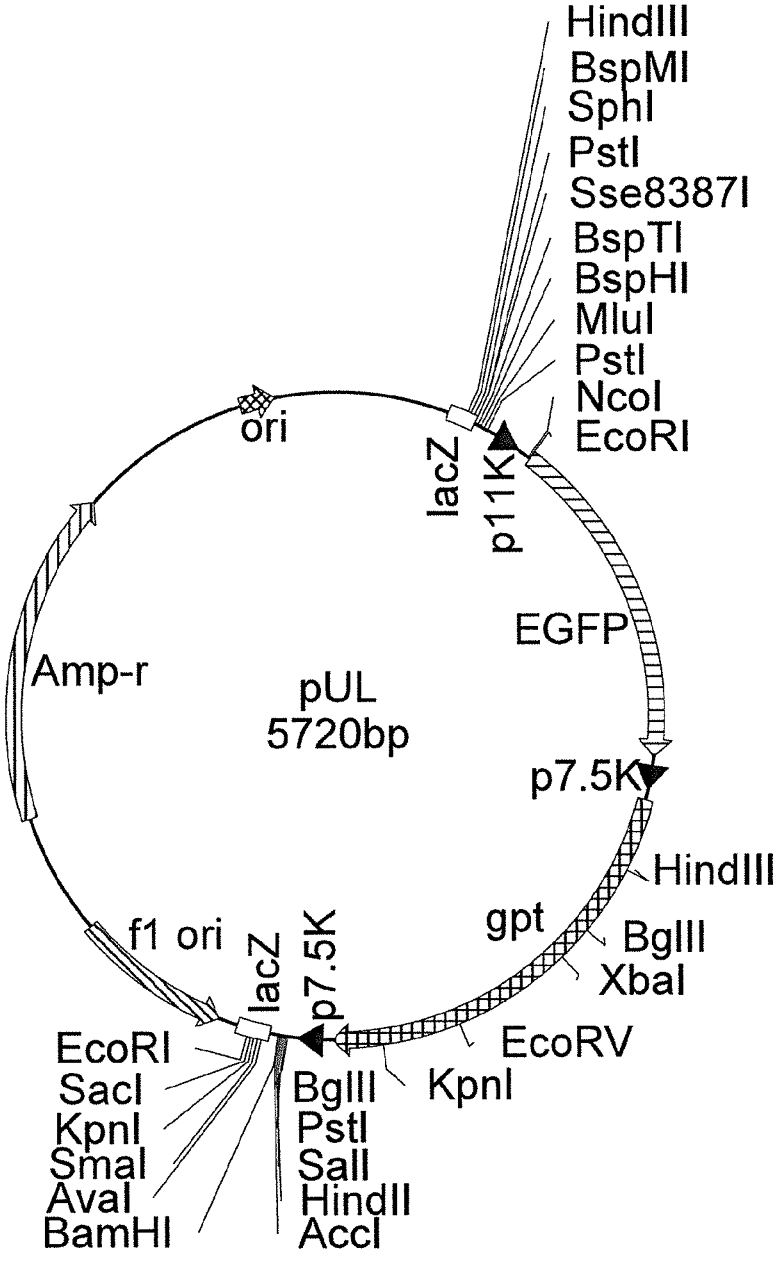
HindIII
BspMI
SphI
PstI
Sse8387I
BspTI
BspHI
MluI
PstI
NcoI
EcoRI
ori
lacZ
p11K
EGFP
Amp-r
pUL
5720bp
p7.5K
HindIII
gpt
BglII
XbaI
f1 ori
lacZ
p7.5K
EcoRV
KpnI
EcoRI
SacI
KpnI
SmaI
AvaI
BamHI
BglII
PstI
SalI
HindII
AccI

**Figure 1.**

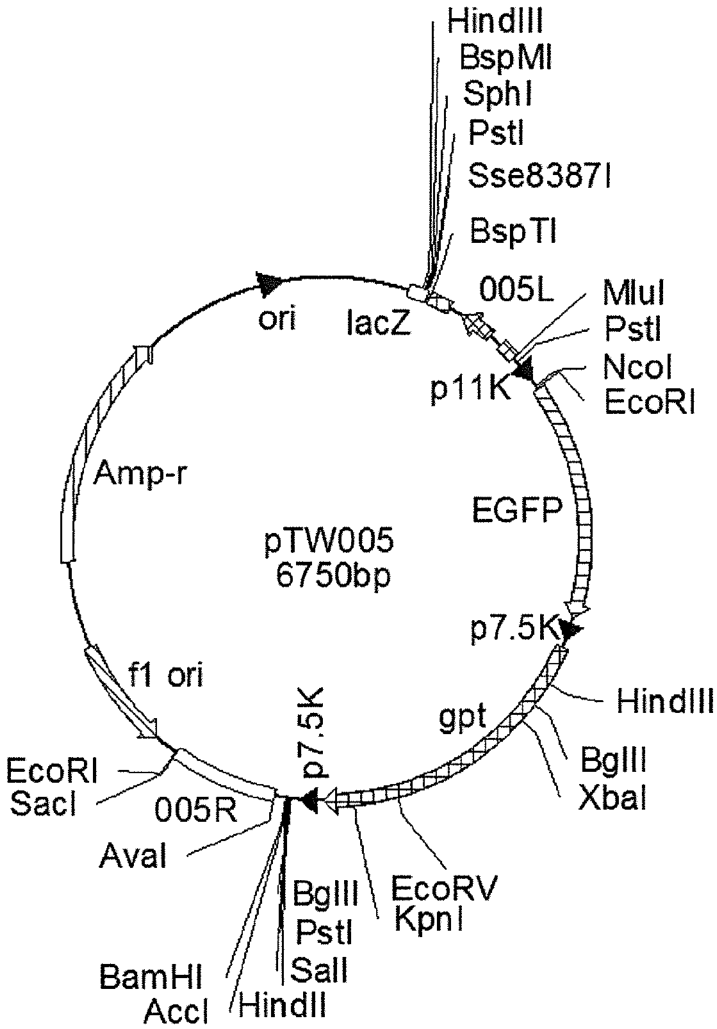
HindIII
BspMI
SphI
PstI
Sse8387I
BspTI
005L
MluI
PstI
NcoI
EcoRI
ori
lacZ
p11K
Amp-r
EGFP
pTW005
6750bp
p7.5K
HindIII
f1 ori
gpt
p7.5K
BglII
XbaI
EcoRI
SacI
005R
AvaI
EcoRV
KpnI
BglII
PstI
SalI
BamHI
AccI
HindII

**Figure 2**

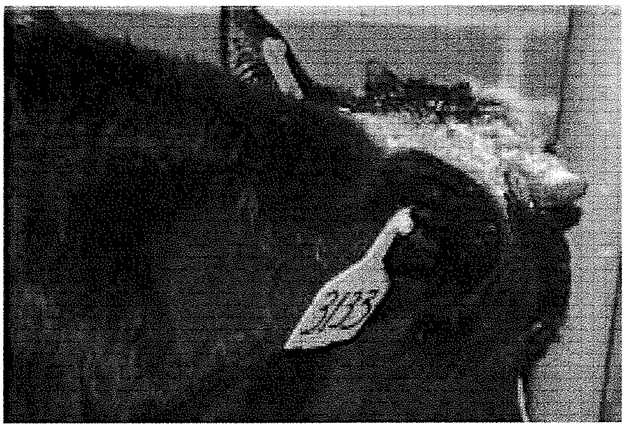

A. Negative control

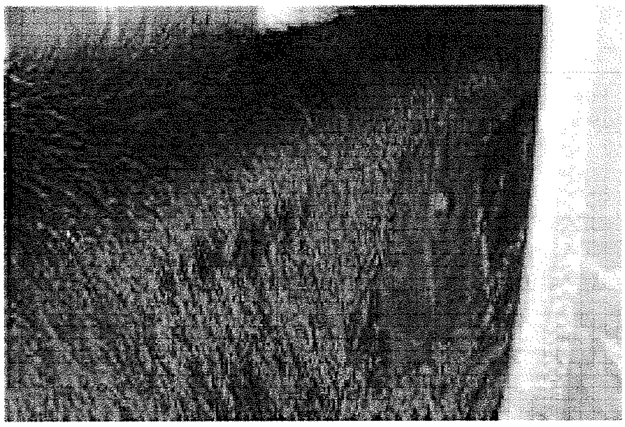

B. LSDV OBP vaccine

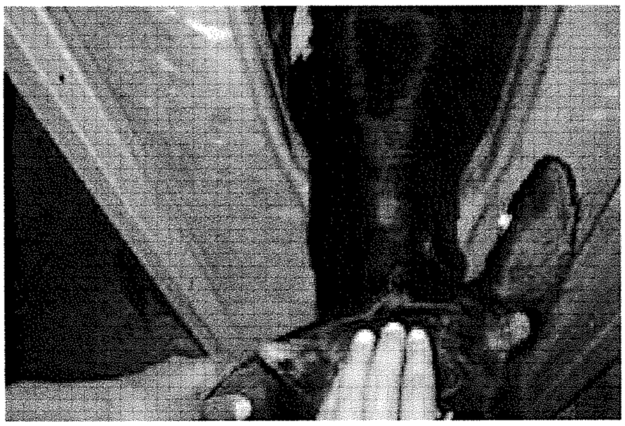

C. IL-10 like gene Knock Out

**Figure 3.**

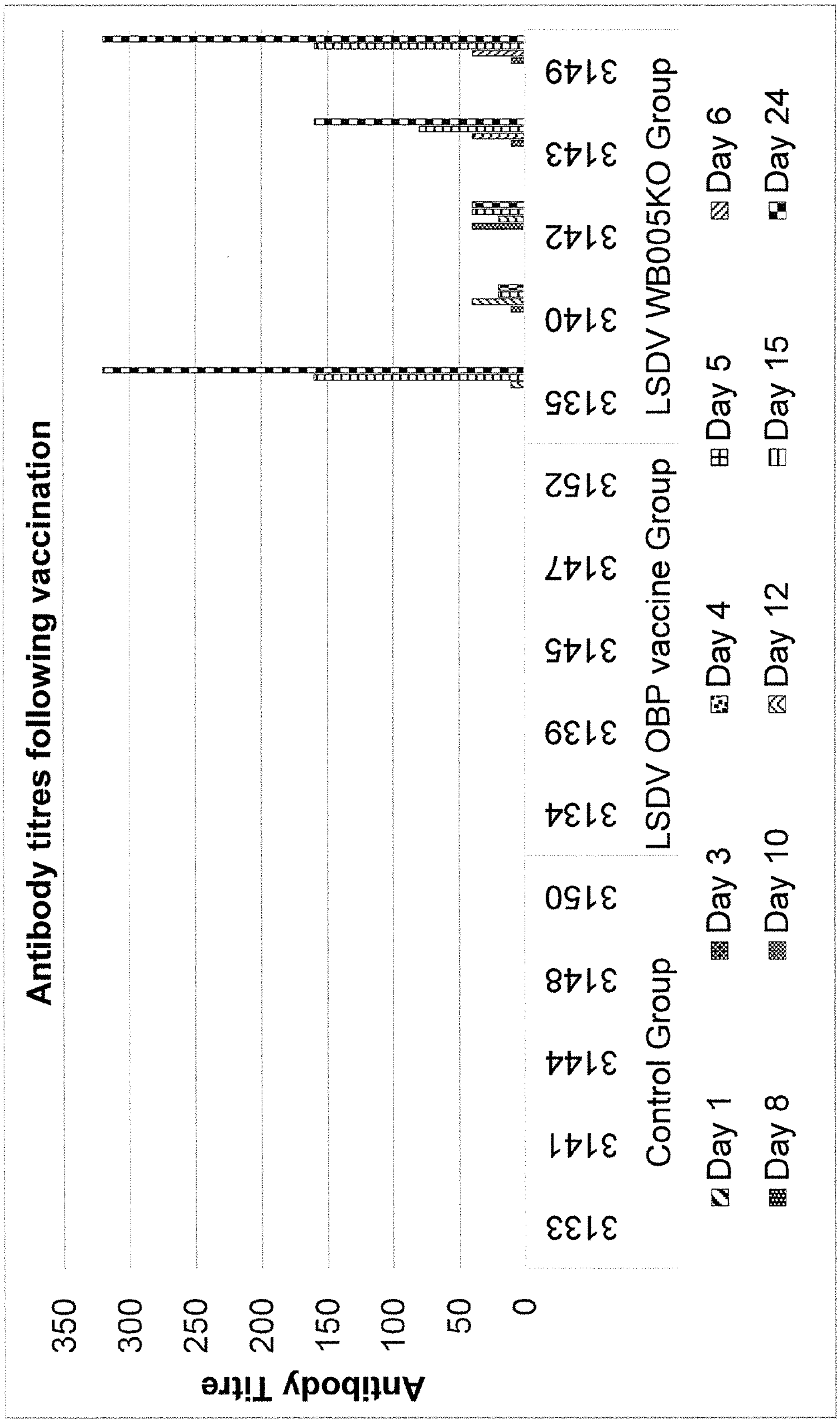
Antibody titres following vaccination
Antibody Titre
350
300
250
200
150
100
50
0
3133
3141
3144
3148
3150
3134
3139
3145
3147
3152
3135
3140
3142
3143
3149
Control Group
LSDV OBP vaccine Group
LSDV WB005KO Group
Day 1
Day 3
Day 4
Day 5
Day 6
Day 8
Day 10
Day 12
Day 15
Day 24

Figure 4

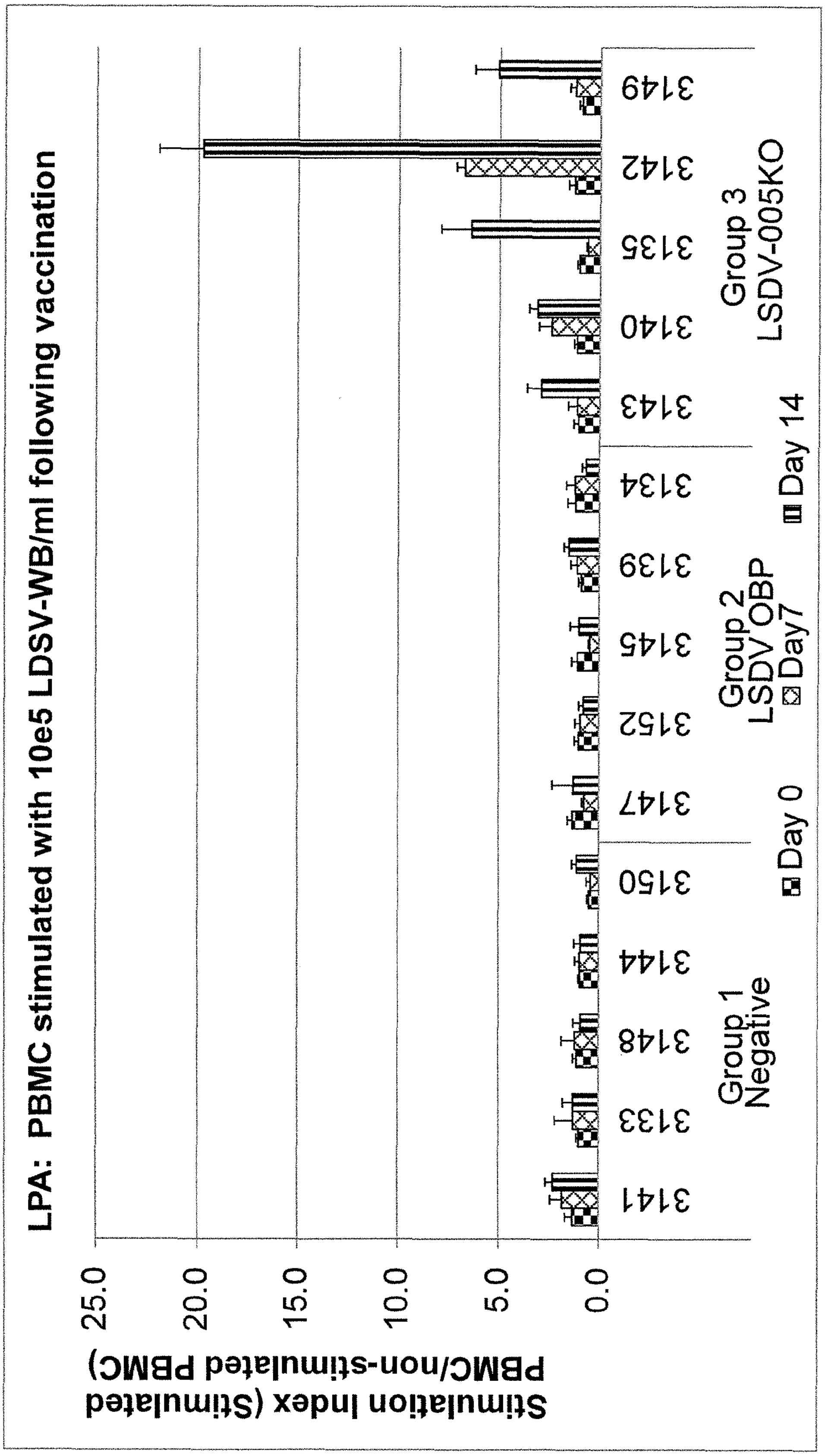
LPA: PBMC stimulated with 10e5 LDSV-WB/ml following vaccination
Stimulation Index (Stimulated PBMC/non-stimulated PBMC)
25.0
20.0
15.0
10.0
5.0
0.0
3141
3133
3148
3144
3150
3147
3152
3145
3139
3134
3143
3140
3135
3142
3149
Group 1 Negative
Group 2 LSDV OBP
Group 3 LSDV-005KO
Day 0
Day7
Day 14

Figure 5

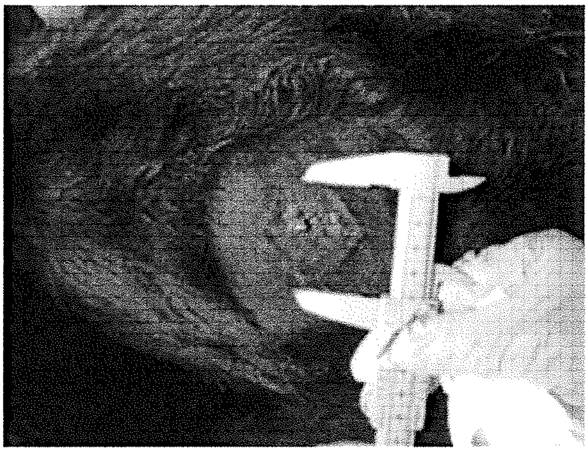

Figure 6

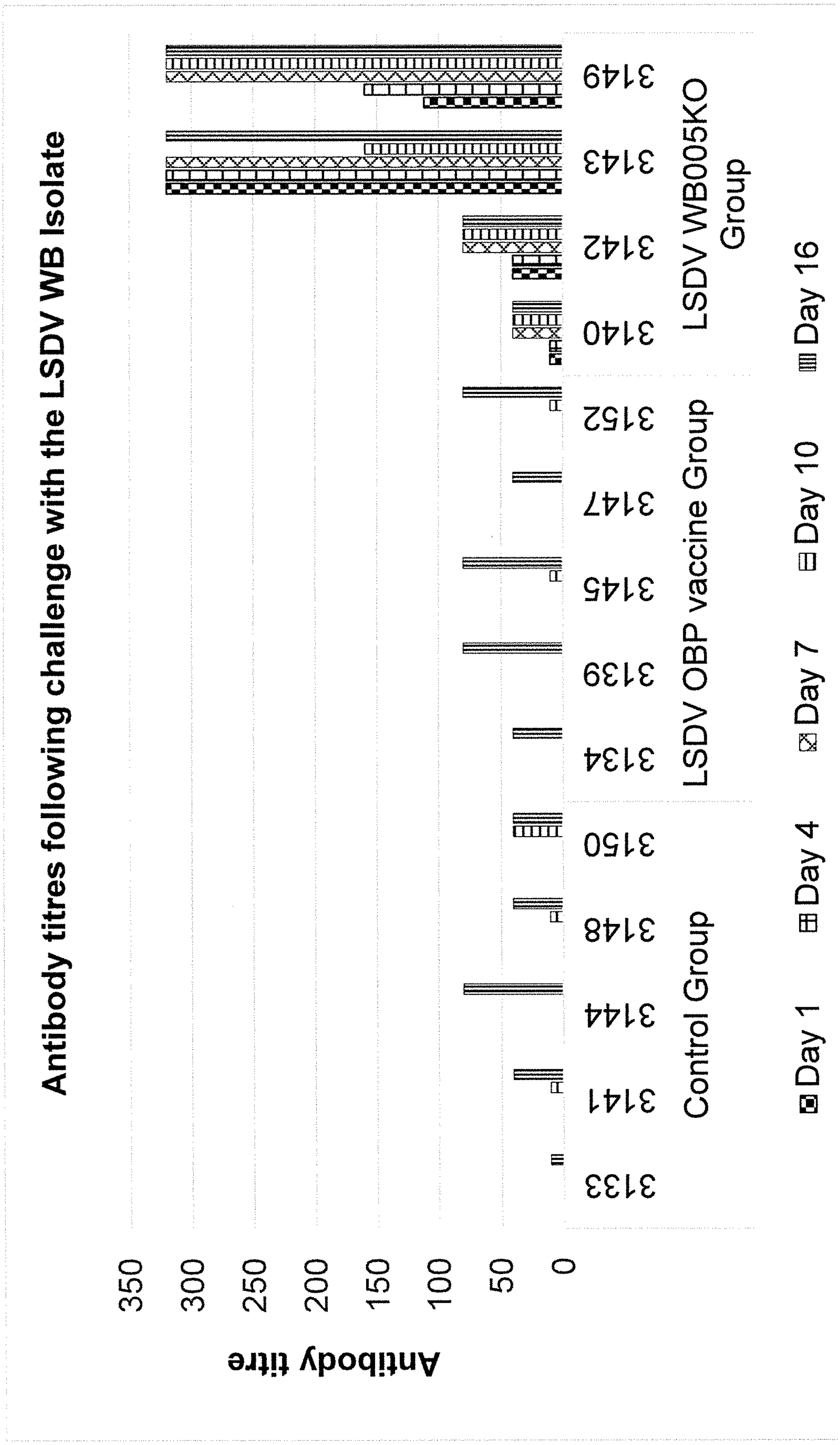

Antibody titres following challenge with the LSDV WB Isolate
Antibody titre
350
300
250
200
150
100
50
0
3133
3141
3144
3148
3150
3134
3139
3145
3147
3152
3140
3142
3143
3149
Control Group
LSDV OBP vaccine Group
LSDV WB005KO Group
Day 1
Day 4
Day 7
Day 10
Day 16


**Figure 7**

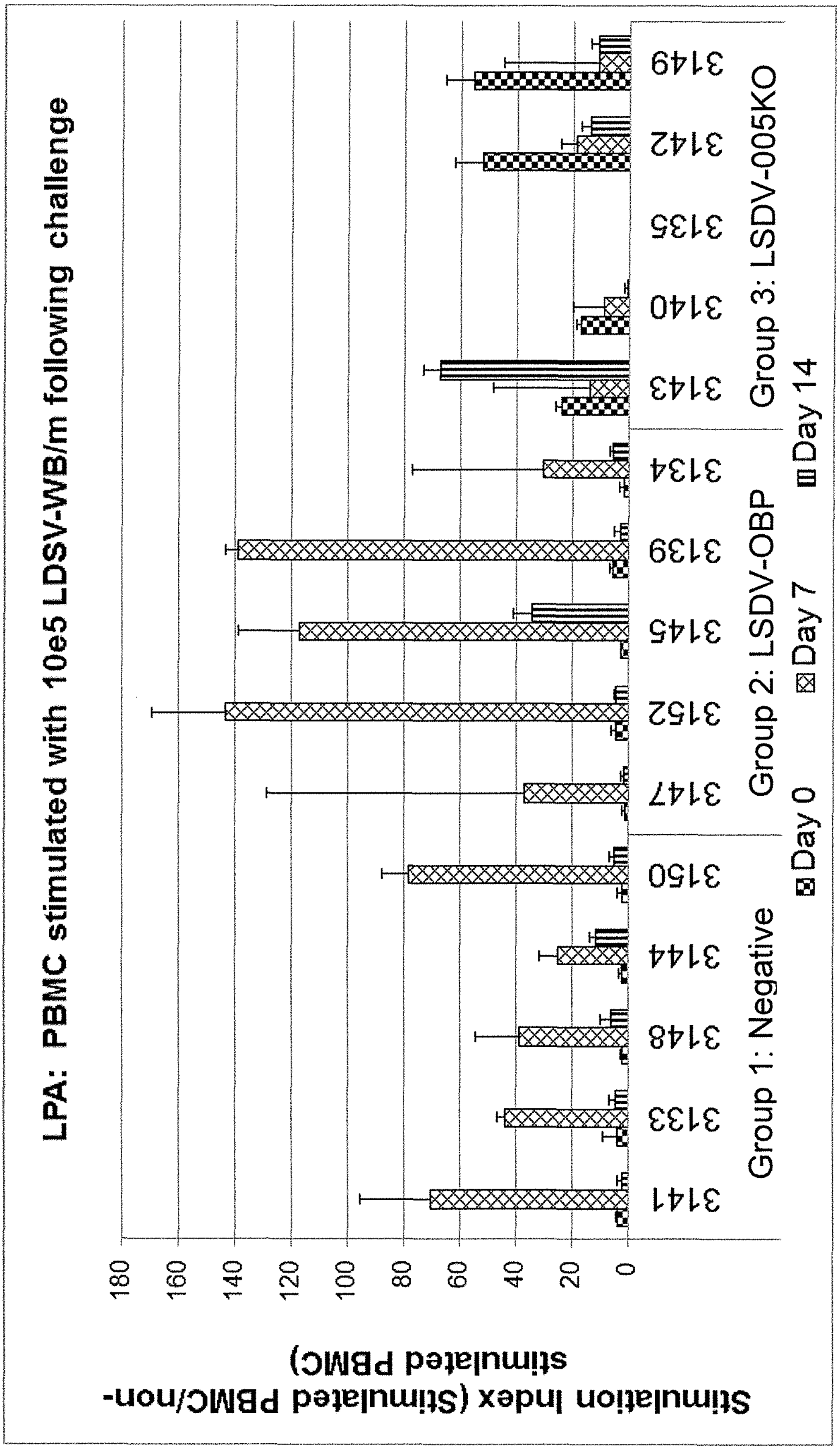
LPA: PBMC stimulated with 10e5 LDSV-WB/m following challenge
Stimulation Index (Stimulated PBMC/non-stimulated PBMC)
180
160
140
120
100
80
60
40
20
0
3141
3133
3148
3144
3150
3147
3152
3145
3139
3134
3143
3140
3135
3142
3149
Group 1: Negative
Group 2: LSDV-OBP
Group 3: LSDV-005KO
Day 0
Day 7
Day 14

Figure 8

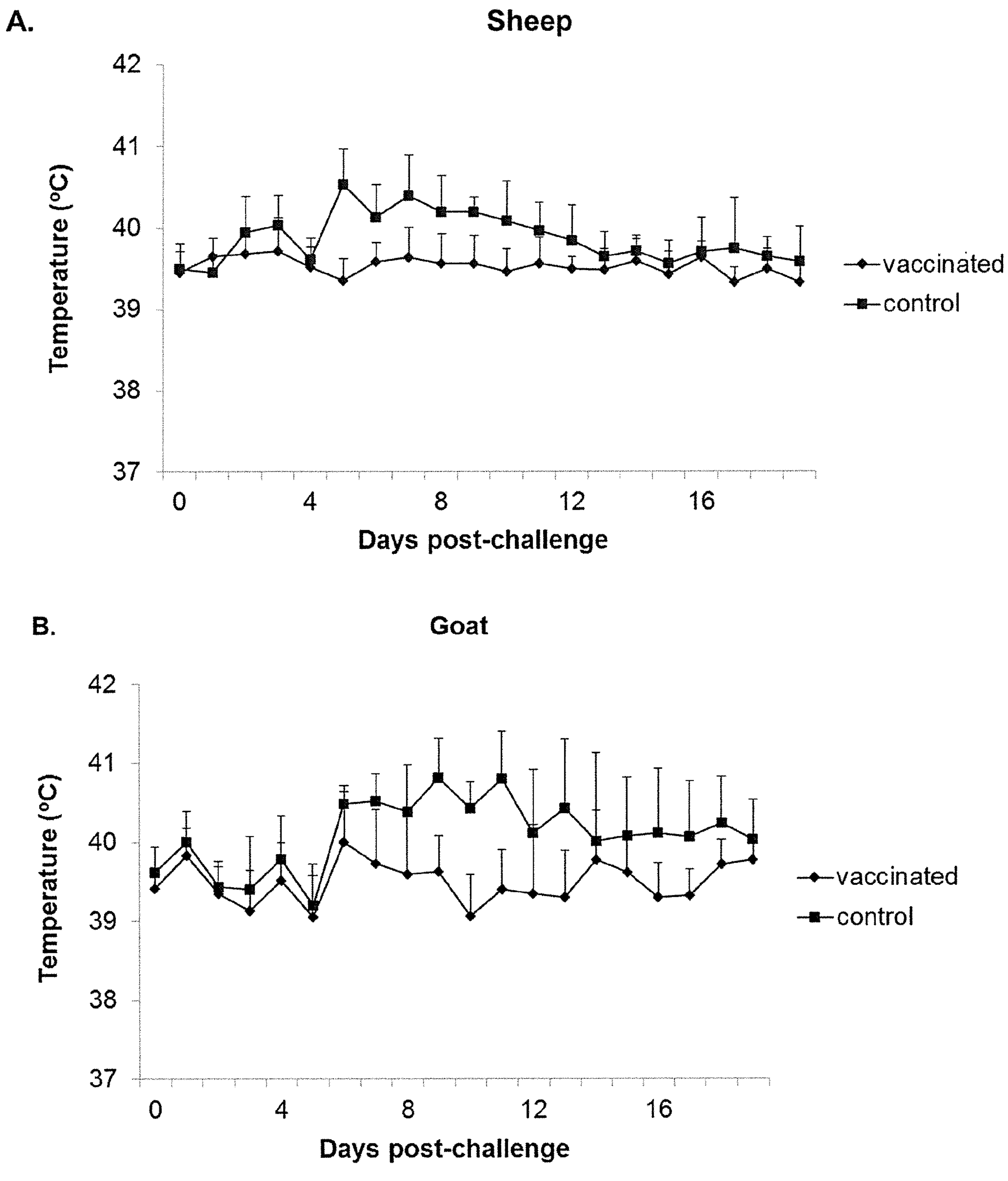
A.
Sheep
42
41
40
39
38
37
Temperature (ºC)
0
4
8
12
16
Days post-challenge
vaccinated
control
B.
Goat
42
41
40
39
38
37
Temperature (ºC)
0
4
8
12
16
Days post-challenge
vaccinated
control

Figure 9

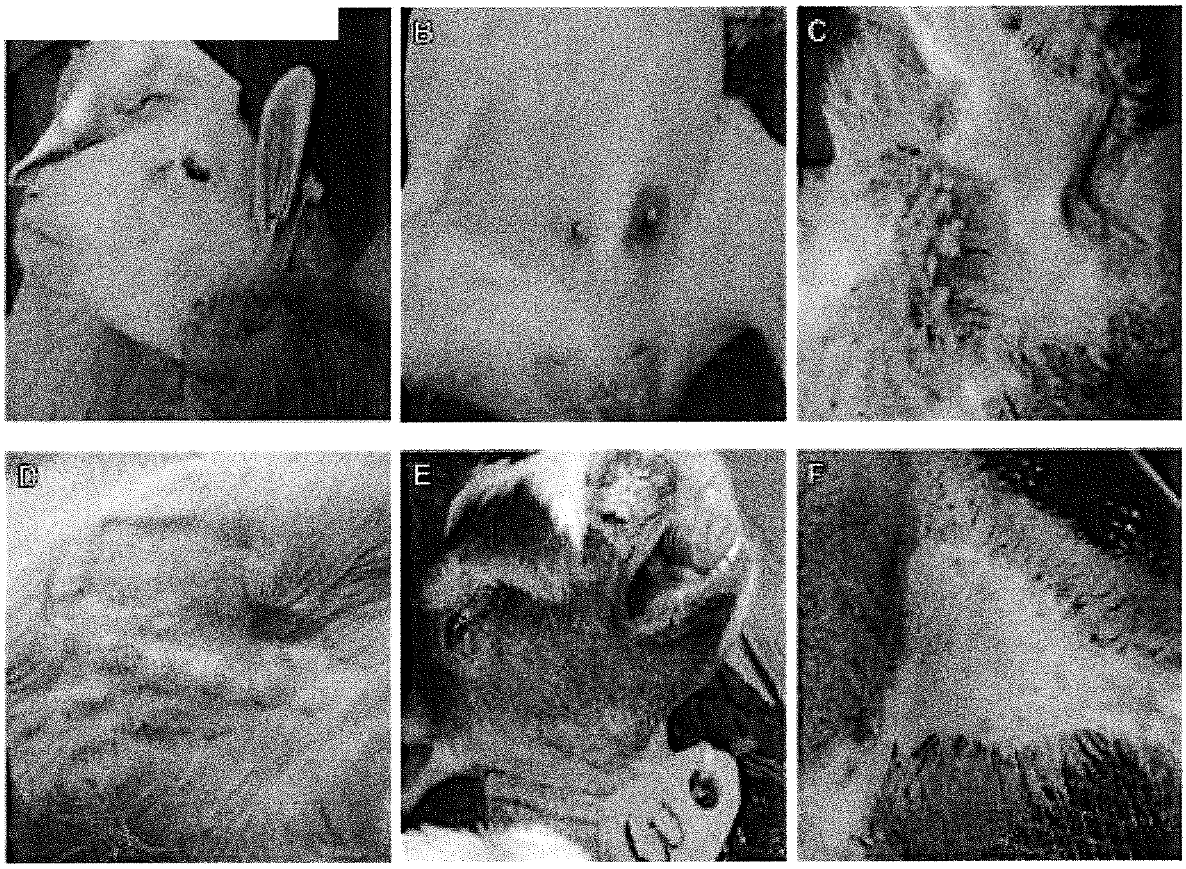


Figure 10

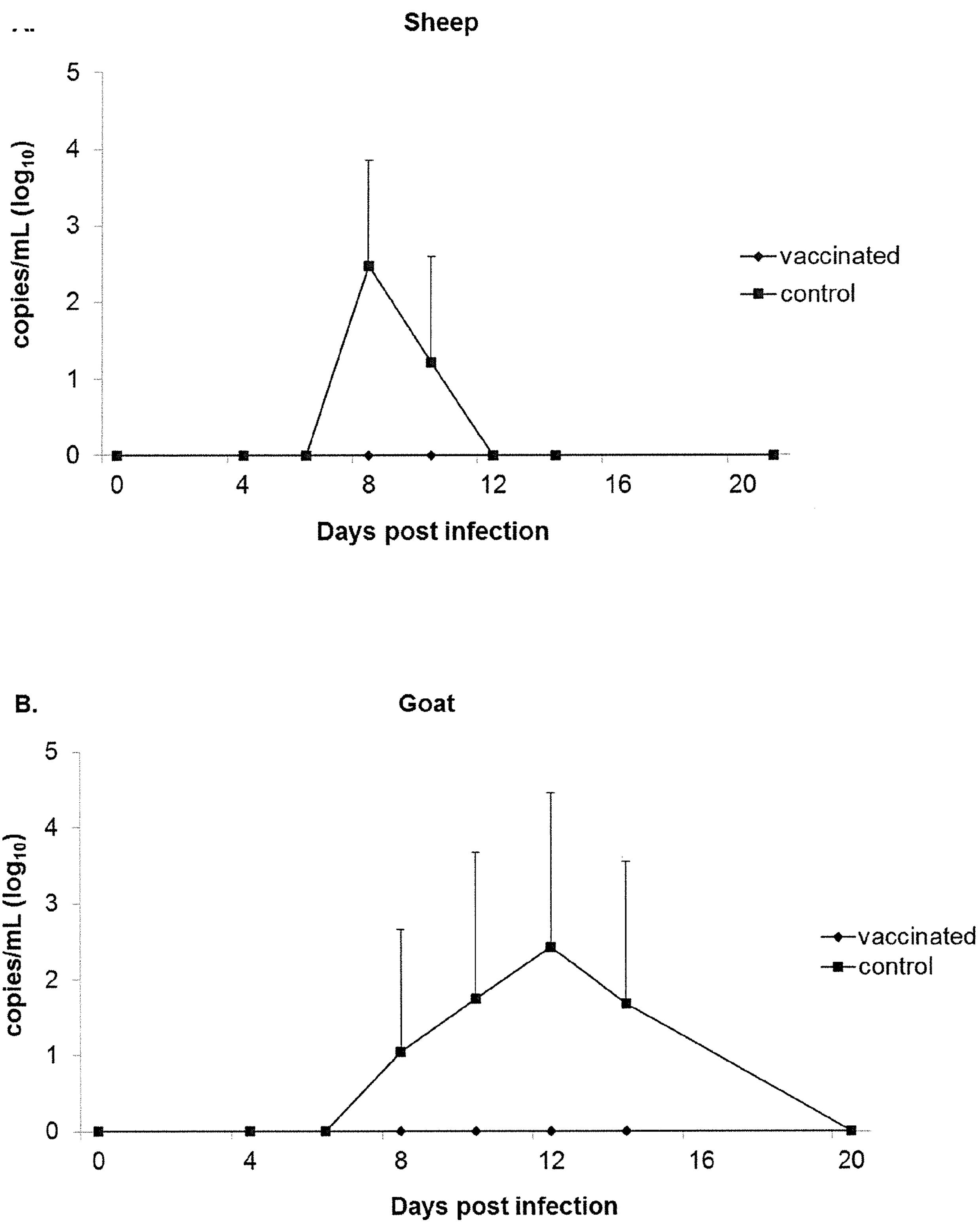
Sheep
copies/mL (log10)
Days post infection
vaccinated
control
B.
Goat
copies/mL (log10)
Days post infection
vaccinated
control

**Figure 11**

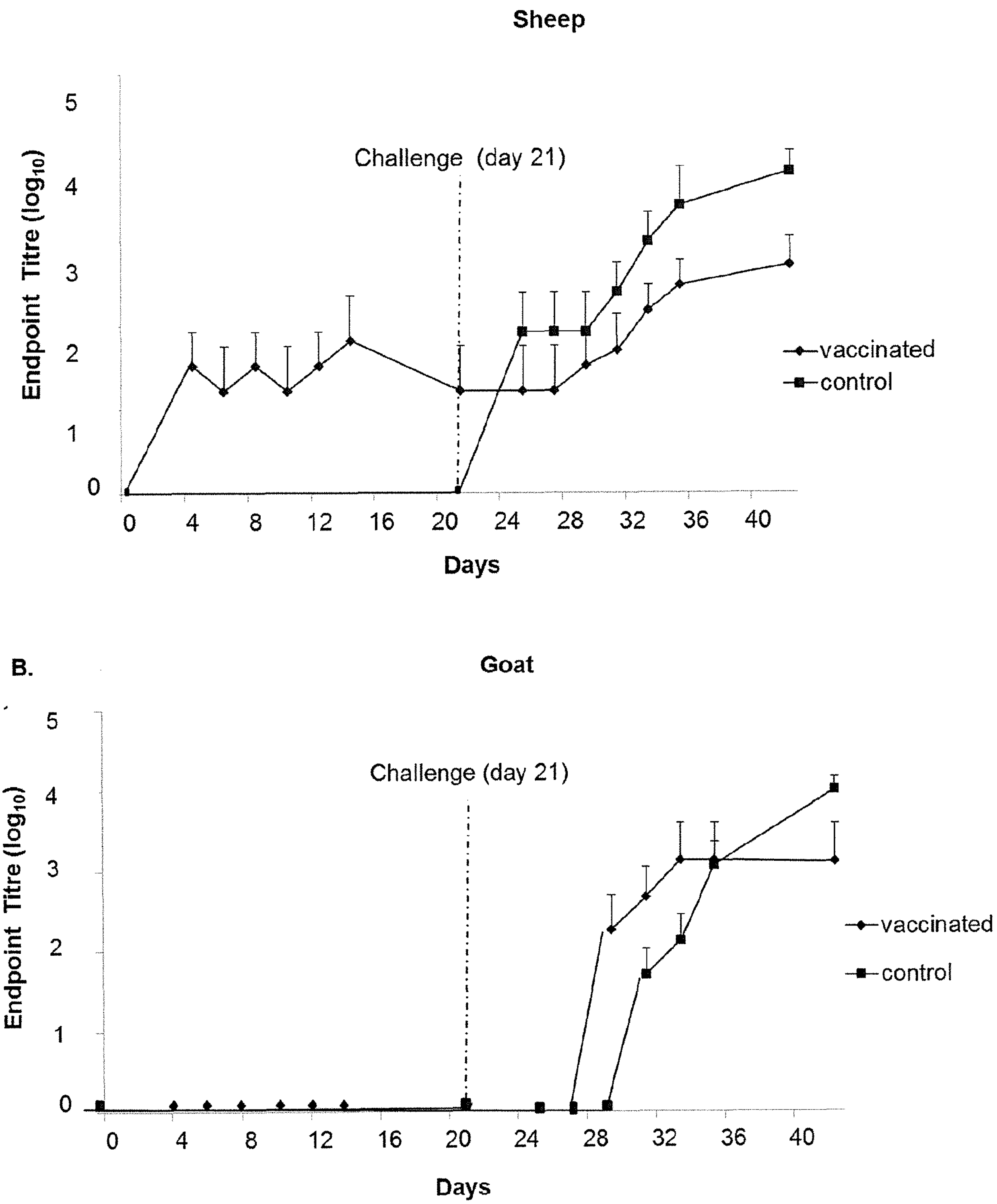
Sheep
Endpoint Titre ($log_{10}$)
Challenge (day 21)
vaccinated
control
Days
B.
Goat
Endpoint Titre ($log_{10}$)
Challenge (day 21)
vaccinated
control
Days

**Figure 12**

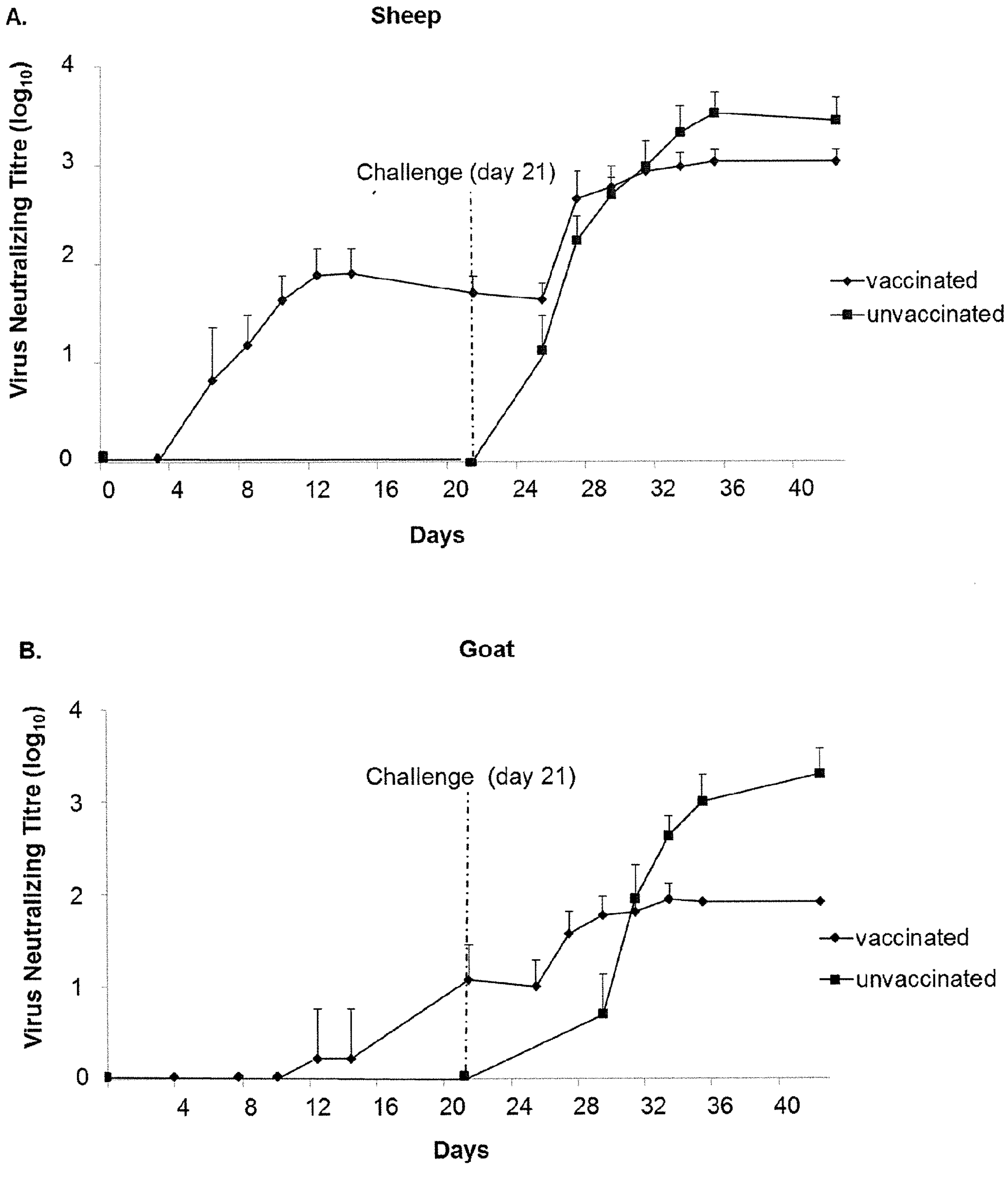
A.
Sheep
Virus Neutralizing Titre (log10)
4
3
2
1
0
Challenge (day 21)
0
4
8
12
16
20
24
28
32
36
40
Days
vaccinated
unvaccinated
B.
Goat
Virus Neutralizing Titre (log10)
4
3
2
1
0
Challenge (day 21)
4
8
12
16
20
24
28
32
36
40
Days
vaccinated
unvaccinated

Figure 13

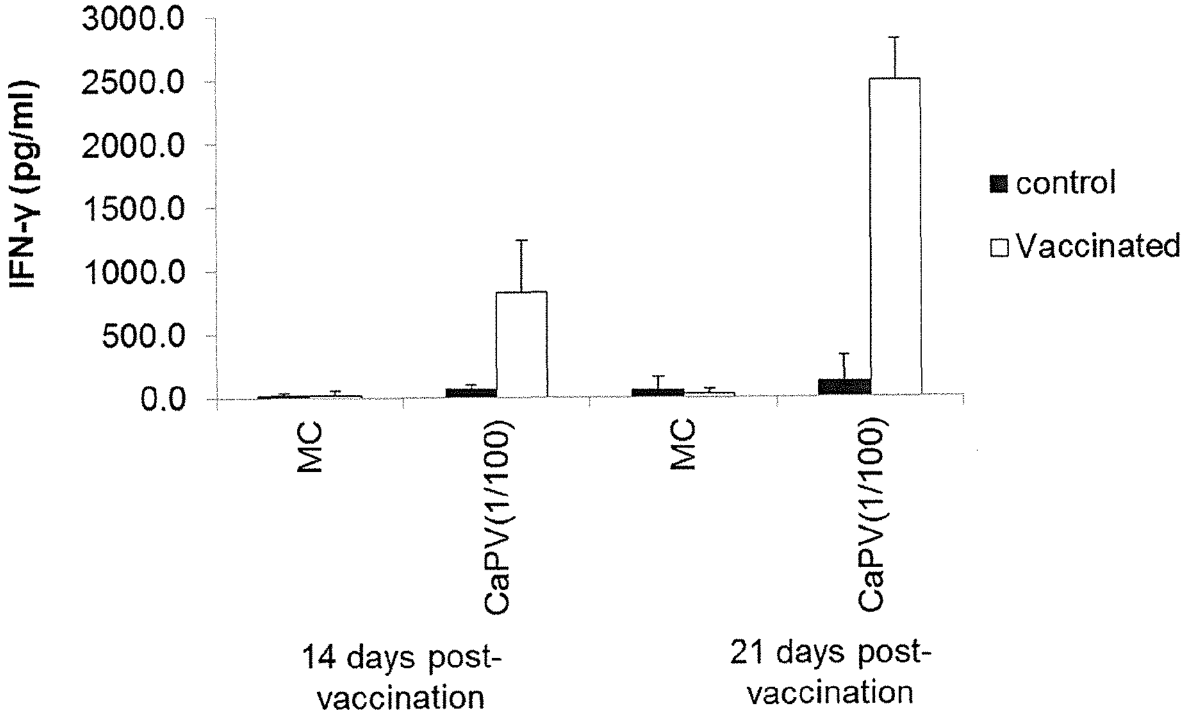
3000.0
2500.0
2000.0
1500.0
1000.0
500.0
0.0
IFN-γ (pg/ml)
control
Vaccinated
MC
CaPV(1/100)
MC
CaPV(1/100)
14 days post-vaccination
21 days post-vaccination

**Figure 14**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KARA P D et al.** Comparative sequence analysis of the South African vaccine strain and two virulent field isolates of Lumpy skin disease virus. *Archives of Virology,* vol. 148, 1335-1356
- **OUYANG P et al.** IL-10 encoded by viruses: a remarkable example of independent acquisition of a cellular gene by viruses and its subsequent evolution in the viral genome. *Journal of General Virology,* vol. 95, 245-262
- **BABIUK, S. et al.** *J. Gen. Virol.,* 2009, vol. 90, 105-114
- **BABIUK, S. et al.** *Transbound Emerg. Dis.,* 2008, vol. 55, 263-272
- **BEARD P.M. et al.** *Veterinary Microbiology,* 2010, vol. 142, 427-431
- **BHANUPRAKASH, V. et al.** *Comp. Immunol. Microbiol. Infect. Dis.,* 2006, vol. 29, 27-60
- **BOWDEN, T.R. et al.** *J. Virol. Methods,* 2009, vol. 161, 19-29
- **BOWDEN, T.R. et al.** *Virology,* 2008, vol. 371, 380-393
- **CAPSTICK, P.B.** *Veterinary Department Annual Report. Government of Kenya,* 1961, 45-47
- **CARO, M.R. et al.** *Res. Vet. Sci.,* 1998, vol. 65, 145-148
- **DAVIES, F.G. ; MBUGWA, G.** *J. Comp. Pathol.,* 1985, vol. 95, 565-572
- **ESPOSITO ; KNIGHT.** *Virology,* 1985, vol. 143 (1), 230-51
- **HUNTER ; WALLACE.** *Journal of the South African Vet Ass,* 2001, vol. 72 (2), 68-71
- **KARA et al.** *Arch Virol.,* 2003, vol. 148 (7), 1335-56
- **KITCHING, R.P.** *Dev. Biol. (Basel),* 2003, vol. 114, 161-167
- OIE, 2010. Sheep And Goat Pox. OIE Terrestrial Manual. 2010, 2.7.14
- *OIE, 2009. Sheep and Goat Pox--World Animal Health/OIE bulletin,* 2009
- **TOURAIS-ESTEVES, I. et al.** *Dev. Comp. Immunol.,* 2008, vol. 32, 1231-1241
- **VAN KLEEF M. et al.** *Infection and Immunity.,* 2000, vol. 68 (2), 603-614
- **VAN ROOYEN, P.J. et al.** *Onderstepoort J. of Vet. Res.,* 1969, vol. 36, 165-174
- **WEISS.** *Virology Monographs,* 1968, vol. 3, 112-282

SEQUENCE LISTING

<110> Agricultural Research Council

<120> Recombinant Lumpy Skin Disease Virus Knock-Out Mutant and Uses Thereof

<130> PA160621/P

<160> 10

<170> PatentIn version 3.5

<210> 1
<211> 52
<212> DNA
<213> Artificial sequence

<220>
<223> Oligonucleotide primer 005-KOL-F

<400> 1
aataaccctg caggcttaag tcatgaatgg aaaacaaaca caaaaataat ac 52

<210> 2
<211> 52
<212> DNA
<213> Artificial sequence

<220>
<223> Oligonucleotide primer 005-KOL-R

<400> 2
taaactgcag acgcgttgat cacttaagca atgtaaaaaa tcacacgata tg 52

<210> 3
<211> 35
<212> DNA
<213> Artificial sequence

<220>
<223> Oligonucleotide primer LF005FWD

<400> 3
atacatctta agcagtttta atcgctctaa aattc 35

<210> 4
<211> 35
<212> DNA
<213> Artificial sequence

<220>
<223> Oligonucleotide primer LF005REV

<400> 4
tagtagacgc gtgtaatcaa agctaacgtc gtcac 35

<210> 5
<211> 38
<212> DNA
<213> Artificial sequence

<220>
<223> Oligonucleotide primer RT005FWD

<400> 5
tcctcccccg gggaaaagta catagttaaa aagtaatc 38

<210> 6
<211> 33
<212> DNA
<213> Artificial sequence

<220>
<223> Oligonucleotide primer RT005REV

<400> 6
atcgagctct atcgtttgta agaaattata acg 33

<210> 7
<211> 18
<212> DNA
<213> Artificial sequence

<220>
<223> Oligonucleotide primer Eco-gpt-F

<400> 7
caggctggga cacttcac 18

<210> 8
<211> 19
<212> DNA
<213> Artificial sequence

<220>
<223> Oligonucleotide primer Eco-gpt-R

<400> 8
gattagcgac cggagattg 19

<210> 9
<211> 667
<212> DNA
<213> Lumpy skin disease virus

<400> 9

```
cccaaaaaaa cttcaaaaat aattatatag agtagtattt accaccaaca tgaaaacaaa        60
cacaaaaata atactattct gttatgttat tcttagtttg tatgtattta gttgtgcaat       120
agcatcggct aaaaaatgtg acgacgttag ctttgattac atattaaaag atttacgttc       180
agaatttagc aaaataaaaa gttttgttca agacaatgat caagaaaata tgatgctatt       240
aagtcaatca atgttggaca agttgacgag tcgcatagga tgtaaatctt tatcggatat       300
gataaaattt tatttaaatg atgttttacc aaatgcagaa aaaatagaac acatgaaaaa       360
taaaataact tcaataggag aaaaattaaa atcgttaaaa gaaaaactca tatcgtgtga       420
ttttttacat tgtgaaaatc atgacgaaat aaaaacagtt aaaacaattt ttaacaaatt       480
aaaagataaa ggtatttata aggctatggg agagtttgat attttcatta attacttaga       540
aaagtacata gttaaaaagt aatcaatcta acttataaaa ctacaacatt atagtttctt       600
tttatgtcat agttaatact atctttgttt attcttaagt tacatgtata aataccacta       660
tcatcat                                                                 667
```

<210> 10
<211> 1547
<212> DNA
<213> Lumpy skin disease virus

<400> 10

```
cccaaaaaaa cttcaaaaat aattatatag agtagtattt accaccaaca tgaaaacaaa      60
cacaaaaata atactattct gttatgttat tcttagtttg tatgtattta gttgtgcaat     120
agcatcggct aaaaaatgtg acgacgttag ctttgattac aacgcgtctg cagctcgagg     180
ccgcgggaat tcgattatag gtctcgagtt tcaggttcag ggggaggtgt gggaggtttt     240
ttaaagcaag taaaacctct acaaatgtgg tatggctgat tatgatcagt tatctagatc     300
cggtggatct gagtccggac ttgtacagct cgtccatgcc gagagtgatc ccggcggcgg     360
tcacgaactc cagcaggacc atgtgatcgc gcttctcgtt ggggtctttg ctcagggcgg     420
actgggtgct caggtagtgg ttgtcgggca gcagcacggg gccgtcgccg atgggggtgt     480
tctgctggta gtggtcggcg agctgcacgc tgccgtcctc gatgttgtgg cggatcttga     540
agttcacctt gatgccgttc ttctgcttgt cggccatgat atagacgttg tggctgttgt     600
agttgtactc cagcttgtgc cccaggatgt tgccgtcctc cttgaagtcg atgcccttca     660
gctcgatgcg gttcaccagg gtgtcgccct cgaacttcac ctcggcgcgg gtcttgtagt     720
tgccgtcgtc cttgaagaag atggtgcgct cctggacgta gccttcgggc atggcggact     780
tgaagaagtc gtgctgcttc atgtggtcgg ggtagcggct gaagcactgc acgccgtagg     840
tcagggtggt cacgagggtg ggccagggca cgggcagctt gccggtggtg cagatgaact     900
tcagggtcag cttgccgtag gtggcatcgc cctcgccctc gccggacacg ctgaacttgt     960
ggccgtttac gtcgccgtcc agctcgacca ggatgggcac caccccggtg aacagctcct    1020
cgcccttgct caccatggct tcggaattca tttatagcat agaaaaaaac aaaatgaaat    1080
tctactatat ttttacatac atatattcta aatatgaaag tggtgattgt gactagcgta    1140
gcactcgagc gcaataatca ctagtgaatt cgcggcctcg agaggcctaa ttaattaagt    1200
cgacttttta tcatatgccc ggtagttgcg atatacataa actgatcact aattccaaac    1260
ccacccactt gttatagtaa gtttttcacc cataaataat aaatacaata attaatttct    1320

cgtaaaagta gaaaatatat tctaatttat tgcacggtaa ggaagtagaa tcataaagaa    1380
cagtcagatc tggatctgca ggtcgacgga tccccgggga aaagtacata gttaaaaagt    1440
aatcaatcta acttataaaa ctacaacatt atagtttctt tttatgtcat agttaatact    1500
atctttgttt attcttaagt tacatgtata aataccacta tcatcat                  1547
```